# EUROPEAN PATENT APPLICATION

(11) **EP 2 615 111 A1**
(43) Date of publication of application: **17.07.2013**
(21) Application number: 12382003.7
(22) Date of filing: 10.01.2012
(51) Int. Cl.: C07K 14/705, C12N 15/62, G01N 33/68, G01N 33/74, G01N 33/542

(54) **Method to identify ligands for sigma-1 receptors**

(71) Applicant: Laboratorios del Dr. Esteve S.A., 08041 Barcelona (ES)
(72) Inventor: Burgueño-Hurtado, Javier, 08820 El Prat de Llobregat Barcelona (ES); Ciruela-Alférez, Francisco, 08328 Alella Barcelona (ES); Vela-Hernandez, José Miguel, 08028 Barcelona (ES)
(74) Representative: ABG Patentes, S.L.

(57) **Abstract**

An assay for identifying ligands of the σ₁ receptor based on a fusion protein comprising the σ₁ receptor flanked by two fluorophores, so that said fluorophores are capable of producing constitutive FRET when no ligand is bound to the σ₁ receptor. The fusion protein of the invention also allows the simultaneous determination whether the newly determined ligand is an agonist or an antagonist. Said fusion protein has been expressed in a cell, giving rise to a new cellular model useful for identifying σ₁ receptor ligands, and additionally for discriminating between agonists and antagonists.

## Description

### TECHNICAL FIELD OF THE INVENTION

The present invention relates to a fusion protein comprising the sigma-1 (σ₁) receptor and two fluorophores and its use in a method for detecting a ligand of the σ₁ receptor using Fluorescence Resonance Energy Transfer (FRET).

### BACKGROUND OF THE INVENTION

The search for new therapeutic agents has been greatly aided in recent years by better understanding of the structure of proteins and other biomolecules associated with target diseases. One important class of these proteins is the sigma (σ) receptor, a cell surface receptor of the central nervous system (CNS) which may be related to the dysphoric, hallucinogenic and cardiac stimulant effects of opioids. From studies of the biology and function of σ receptors, evidence has been presented that σ receptor ligands may be useful in the treatment of psychosis and movement disorders such as dystonia and tardive dyskinesia, and motor disturbances associated with Huntington's chorea or Tourette's syndrome and in Parkinson's disease (Walker, J.M. et al. 1990, Pharmacol. Rev. 42, 355). It has been reported that the known σ receptor ligand rimcazole clinically shows effects in the treatment of psycosis (Snyder, S.H. and Largent, B.L. 1989, J. Neuropsychiatry 1, 7). The σ binding sites have preferential affinity for the dextrorotatory isomers of certain opiate benzomorphans, such as (+)SKF-10,047, (+)cyclazocine, and (+)pentazocine and also for some narcoleptics such as haloperidol.

The σ receptor has at least two subtypes, which may be discriminated by stereoselective isomers of these pharmacoactive drugs. SKF-10,047 has nanomolar affinity for the σ₁ site and micromolar affinity for the sigma-2 (σ₂) site. Haloperidol has similar affinities for both subtypes. Endogenous σ ligands are not known, although progesterone has been suggested to be one. Possible σ-site-mediated drug effects include modulation of glutamate receptor function, neurotransmitter response, neuroprotection, behaviour, and cognition (Quirion, R. et al. 1992, Trends Pharmacol. Sci. 13, 85-86). Most studies have implied that σ binding sites (receptors) are plasmalemmal elements of the signal transduction cascade. Drugs reported to be selective σ ligands have been evaluated as antipsychotics (Hanner, M. et al. 1996, Proc. Natl. Acad. Sci. USA 93, 8072-8077). The existence of σ receptors in the CNS, immune and endocrine systems suggests that they may serve as link between the three systems.

To date, clear in vitro demonstration of functional properties by σ ligands remain inconclusive. Nevertheless, in the literature published so far ligands such as (+)-3-PPP, dextromethorphan, (+)-SKF-10,047, PRE-084, and (+)-pentazocine are considered to be agonist at the σ₁ receptor whereas ligands such as haloperidol, BD-1063, BD-1047, NE-100, ditolyl guanidine (DTG) and progesterone are considered to be antagonists (Cobos et al. 2005, Synapse 55,192-195).

The σ₁ receptor is expressed in areas important for pain control such as the spinal cord, the periaqueductal grey matter and the rostroventral medulla. In the spinal cord it is expressed mainly in the two superficial laminae of the dorsal horn, in dendritic processes and neuronal perikarya, where it is localized both on the plasma and the endoplasmic reticulum (Alonso G. et al. 2000, Neuroscience 97, 155-170). Interestingly, σ₁ receptor expression in the spinal cord is upregulated during the induction phase of neuropathic pain (Roh D.H. et al. 2008, Anesthesiology 109, 879-889). The unparalleled ability of σ₁ receptors to interact with a huge range of drug structural classes and its wide distribution in the body has contributed to it being implicated as a possible therapeutic target for a broad array of disorders, including cancer, depression, psychosis, substance abuse, Alzheimer's disease, cerebral stroke, and other traumatic brain injuries.

In view of the potential therapeutic applications of agonists or antagonists of the σ₁ receptor, a great effort has been made to find selective ligands. Methods for this purpose are already known in the art:
For example, US patent application n° 2009017038 discloses methods of screening and identifying novel ligands specific for the σ₂ receptor, in which candidate compounds are contacted with one or more human histones involved in the σ₂ receptor function.

Japanese patent application n° 2009102306 discloses techniques for screening a compound capable of specifically binding to a σ₁ receptor comprising a fusion protein having a molecular chaperone activity or its subunit having ligand binding activity and σ₁ receptor.

WO2010059711 describes a method to identify selective σ₁ receptor ligands by competitive displacement of radioactive pentazocine. For determination of binding to the σ₂ receptor, radioactive DTG was utilized in the presence of non-radioactive pentazocine, which masked the σ₁ receptor population from binding to DTG. Nonspecific binding was determined by adding Haloperidol as a control condition.

σ₁ receptor ligands can be classified as agonist or antagonist with an assay *in vivo.* Specifically, on capsaicin-induced mechanical hypersensitivity, σ₁ receptor antagonists can reduce capsaicin-induced pain whereas agonists potentiate pain by a sub-threshold capsaicin dose (Brenchat A. et al. 2011, Eur. J. Pain, in press**;** Entrena J.M. et al. 2009, Pain 143, 252-261). Even more, in this pain model the effects produced by antagonist can be reversed by the agonist and the other way round. Although very useful to characterize the functionality of σ₁ receptor ligands, these *in vivo* assays are very time consuming and lack the higher throughput of *in vitro* assays making them inappropriate for screening high number of compounds.

Generally, it would be desirable to investigate ligand-receptor interaction *in vivo* by non-invasive techniques that can help to validate the physiological significance of the interaction and to identify the receptor-ligand binding. Also, it would be desirable to have screening methods that allow for fast and reliable screening and selection of compounds that are ligands of the σ receptor, and especially those that interact with the receptor in the context of a living cell. It would also be of high interest if the methods can discriminate between the different ligands of the σ receptor: agonists and antagonists.

Therefore, there is a need in the art for alternative methods or assays to identify novel σ₁ receptor ligands useful in the treatment of σ₁ receptor mediated diseases or conditions. As a consequence, it is an object of the present invention to provide an improved method of selecting σ₁ receptor ligands.

### SUMMARY OF THE INVENTION

In a first aspect, the present invention relates to a fusion protein comprising
i) a σ₁ receptor or a functionally equivalent variant thereof,
ii) a donor fluorescent protein moiety, and
iii)an acceptor fluorescent protein moiety,
wherein said donor and acceptor fluorescent protein moiety are capable of producing Fluorescence Resonance Energy Transfer (FRET), and wherein the σ₁ receptor is flanked by the donor fluorescent protein moiety and the acceptor fluorescent protein moiety.

In a second aspect, the present invention relates to polynucleotides comprising a coding sequence encoding a fusion protein according to the first aspect.

In a third aspect, the present invention relates to a cell comprising a membrane comprising the fusion protein of the first aspect, the fusion protein of the first aspect and/or a polynucleotide of the second aspect, as well as to the use of said cell.

In a fourth aspect, the present invention relates to a cell-free or a cell-based system using the fusion protein of the first aspect to identify ligands of the σ₁ receptor. More specifically, it relates to a method for identifying a ligand for a σ₁ receptor comprising:
(i) exposing a fusion protein according to the first aspect, a fusion protein comprised in a membrane according to the first aspect, or a fusion protein comprised in a cell according to the second aspect to light of a wavelength exciting the donor fluorescent protein moiety, upon which the donor fluorescent protein moiety fluoresces at a first emission wavelength, wherein the light of the first emission length is capable of exciting the acceptor fluorescent protein moiety;
(ii) detecting light of a second emission wavelength emitted by the acceptor protein moiety;
(iii) contacting the fusion protein, membrane and/or cell of step (i) with a test compound; and
(iv) detecting light of a second emission wavelength emitted by the acceptor protein moiety of the fusion protein of step (iii) and, optionally, a control;
   wherein, if the intensity of light of a second emission wavelength emitted during step (i) is known or if a control is included, step (ii) may be omitted and, when omitted, step (iii) may be carried out before step (i), and wherein the test compound is identified as a ligand for a σ₁ receptor if there is a difference in the intensity of light of the second emission wavelength detected in step (iv) and
   - the intensity of light of the second emission wavelength detected in step (ii),
   - the intensity of light of the second emission wavelength known to be emitted during step (i), or
   - the intensity of light of the second emission wavelength of the control.

### BRIEF DESCRIPTION OF THE FIGURES

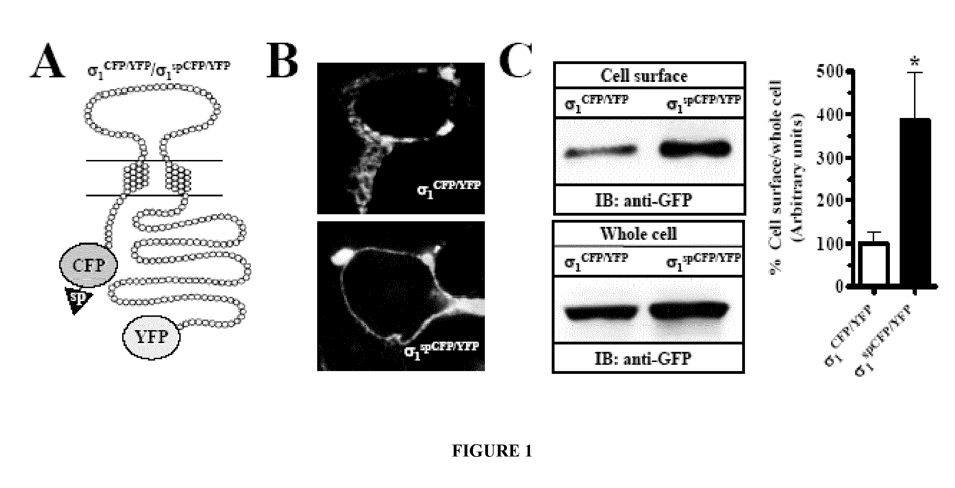

**Figure 1****. Biochemical characterization of the σ₁ constructs transiently transfected in HEK-293 cells. A.** Overall transmembrane topology of the σ₁ FRET sensor constructs (i.e. σ₁^{CFP/YFP} and σ₁^{spCFP/YFP})_{.} **B.** Fluorescence detection of the σ₁ constructs. **C**. Cell surface detection of σ₁ constructs by means of biotinylation experiments. The right panel shows the quantification of four independent biotinylation experiments. The asterisk denote the statistical significance differences (*P<0.05; Student t test).

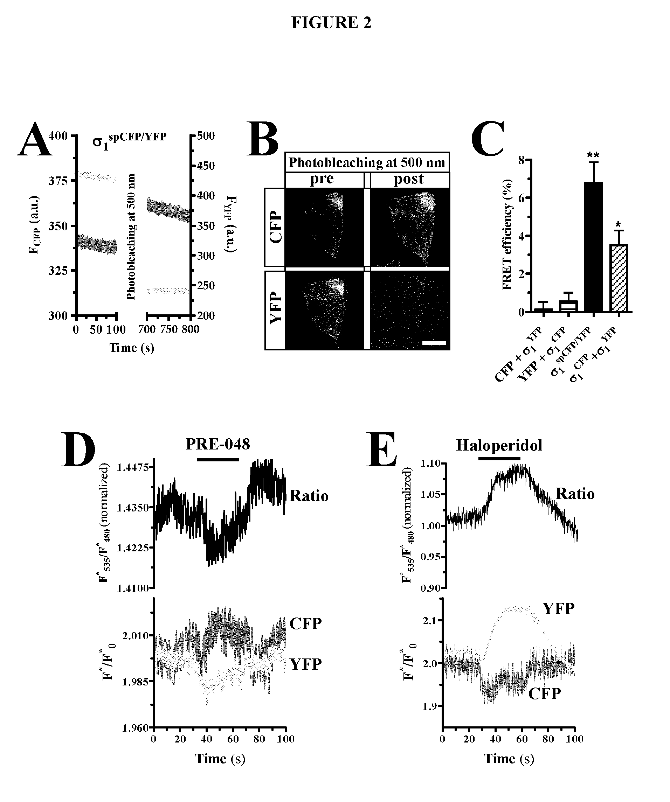

**Figure 2****. FRET efficiency and time-resolved changes in the FRET signal of** σ₁^{spCFP/YFP}. **A**. Effect of photobleaching on the σ₁^{spCFP/YFP} construct fluorescence profile. **B**. Fluorescence detection of the σ₁^{spCFP/YFP} construct before (pre) and after (post) photobleaching. **C**. Quantification of the FRET efficiency of different FRET pairs: CFP plus σ₁^{YFP} (n=5), YFP plus σ₁^{CFP} (n=7), σ_{1,}^{spCFP/YFP} (n=15), and σ₁^{CFP} plus σ₁^{YFP} (n=7)_{.} Data indicate mean ± s.e.m. and the asterisks denote the statistical significance differences versus the CFP+σ₁^{YFP} group (*P<0.05 and **P<0.01; Student t test). **D-E.** σ₁ receptor agonist (100 µM PRE-48; panel D) and antagonist (100 µM haloperidol; panel E)-mediated changes in the F^{*}₅₃₅/F^{*}₄₈₀ ratio of σ₁^{spCFP/YFP} construct expressed in HEK-293 .

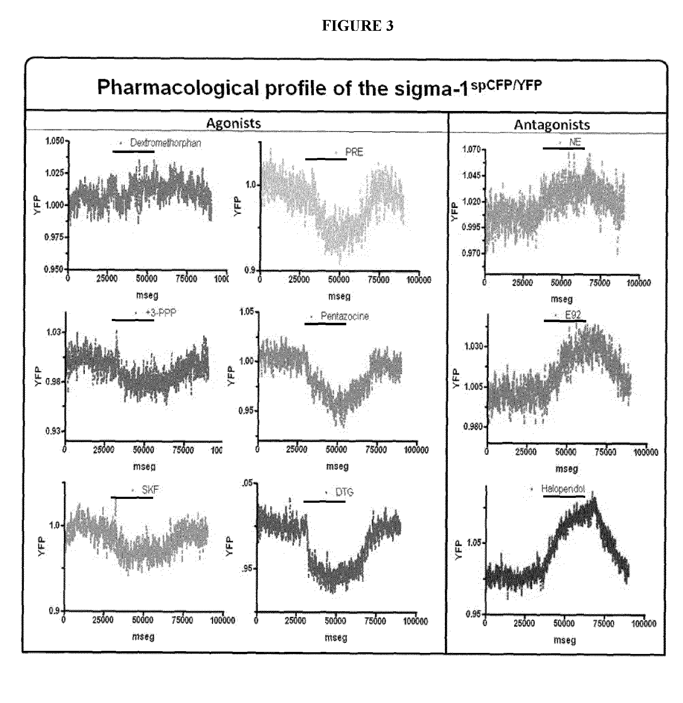

**Figure 3****. Pharmacological profile of the σ₁^{spCFP/YFP} construct.** The σ₁^{spCFP/YFP} construct expressed in HEK-293 cells is incubated with 100 µM of σ₁ receptor agonists (Dextromethorphan, (+)-3-PPP, (+)-Pentazocine, (+)-SKF-10,047, DTG and PRE-048) and antagonists (Haloperidol, E92 and NE-100) and the changes in the F^{*}₅₃₅/F^{*}₄₈₀ ratio determined by single-cell real-time intramolecular-FRET.

### DETAILED DESCRIPTION OF THE INVENTION

The present invention is based on the inventors' studies regarding the σ₁ receptor functionality. The inventors aimed at employing the FRET-technology for identifying novel σ₁ receptor ligands. FRET (Fluorescence Resonance Energy Transfer, or, in case only fluorescent molecules are used, Fluorescent Resonance Energy Transfer) is a mechanism of energy transfer between two fluorophores. A donor fluorophore, in its electronic excited state, may transfer energy to an acceptor fluorophore in proximity through nonradiative dipole-dipole coupling. FRET is a useful tool to quantify molecular dynamics, such as protein-protein interactions, protein-DNA interactions, and protein conformational changes.

For example, cyclic AMP can be detected by FRET between separately labeled proteins that associate with each other but are not covalently attached to each other (see, U.S. Pat. No. 5,439,797). Likewise, calcium levels can be detected using a fluorescent indicator that includes a binding protein moiety, a donor fluorescent protein moiety, and an acceptor fluorescent protein moiety. The binding protein moiety has an analyte-binding region which binds an analyte and causes the indicator to change conformation upon exposure to the analyte. The donor fluorescent protein moiety is covalently coupled to the binding protein moiety. The acceptor fluorescent protein moiety is also covalently coupled to the binding protein moiety. In the fluorescent indicator, the donor moiety and the acceptor moiety change position relative to each other when the analyte binds to the analyte-binding region, altering FRET between the donor moiety and the acceptor moiety when the donor moiety is excited (see U.S. Pat. No. 6,197,928).

As is apparent, the FRET technology is not necessarily useful for detecting conformational changes in any protein. For FRET to be functional, donor and acceptor fluorophores fused to the protein to be studied should be sufficiently distant in the non-excited state and then, the conformational change must bring both fluorophores in close enough proximity so that the donor fluorophore can excite the acceptor fluorophore. Also, in case of, for example, a receptor to be studied, receptors may not aggregate in a way that the donor fluorophore of one receptor and the acceptor fluorophores of another receptor are brought into close proximity. Furthermore, the fusion of the two fluorophores to the protein to be studied may disturb its conformation or its conformational change, rendering it non-functional, e.g. with respect to ligand binding.

Overcoming these difficulties, the inventors have succeeded in developing an assay for identifying ligands of the σ₁ receptor based on a fusion protein comprising the σ₁ receptor flanked by two fluorophores, so that said fluorophores are capable of producing constitutive FRET when no ligand is bound to the σ₁ receptor. Surprisingly, this phenomenon can be altered in a ligand-type specific fashion: if an agonist binds to the σ₁ receptor, the intensity of light emitted by the acceptor fluorophore (i.e. FRET) is decreased, and if an antagonist binds to the receptor, the intensity of light emitted by the acceptor fluorophore (i.e. FRET) is increased. Thus, the fusion protein of the invention does not only allow the identification of σ₁ receptor ligands, but also the simultaneous determination whether the newly determined ligand is an agonist or an antagonist. Said fusion protein has been expressed in a cell, giving rise to a new cellular model extremely useful for identifying σ₁ receptor ligands, and additionally for discriminating between agonists and antagonists.

Based on this, the inventors have developed a series of inventive aspects that are described in detail below.

### FUSION PROTEIN OF THE INVENTION

In a first aspect, the present invention relates to a fusion protein comprising
i) a σ₁ receptor or a functionally equivalent variant thereof,
ii) a donor fluorescent protein moiety, and
iii)an acceptor fluorescent protein moiety,
wherein said donor and acceptor fluorescent protein moiety are capable of producing Fluorescence Resonance Energy Transfer (FRET), and wherein the σ₁ receptor is flanked by the donor fluorescent protein moiety and the acceptor fluorescent protein moiety.

The term "sigma-1 receptor", "sigmar1" or "σ1 receptor" as used herein relates to a ligand-regulated molecular chaperone, which modulates intracellular signalling cascades incurred when the target protein it is interacting with becomes activated. As a ligand-regulated chaperone, the modulatory activity on the target protein can be enhanced or inhibited by agonists or antagonists acting on σ₁ receptors. Notably, under normal physiological conditions, most target proteins are not affected by σ₁ receptor ligands. Only when disturbed or stressed can specific targeted ion channels or receptors be assisted by σ₁ receptor chaperones, allowing σ₁ receptor ligands to exert modulatory effects. The σ₁ receptor to be used according to the present invention is not limited to any species and may be derived, for example, from rat, mouse, frog, zebra fish, chimpanzee, Rhesus monkey, guinea pig, cow, horse, or human. In a preferred embodiment, the σ₁ receptor to be used according to the present invention is the human σ₁ receptor (NCBI reference number NM_005866).

The fusion protein of the first aspect may also comprise functionally equivalent variants of the σ₁ receptor. The term "functionally equivalent variant" as used herein is to be understood to include all those amino acid sequences derived from the σ₁ receptor amino acid sequence by means of modifications or mutations, including substitutions, preferably conservative substitutions, insertions and/or deletions, affecting one or more amino acids, provided that the function of the σ₁ receptor is substantially maintained. Said function can be the modulation of intracellular signalling cascades incurred when a target protein the σ₁ receptor is interacting with becomes activated. Alternatively, it may also be the ability of binding known ligands, for example those described by Lee and collaborators (Lee et al. 2008, Eur. J. Pharmacol. 578, 2-3), e.g. haloperidol. Suitable assays for determining if a function of the σ₁ receptor is substantially maintained are widely known and are described in the prior art, e.g. competition assays using the radioligand [³H]-(+)-pentazocine (see, e.g., Lee et al. 2008, Eur. J. Pharmacol. 578, 2-3). The term "substantially maintained" preferably refers to the ability of binding known antagonist ligands, (e.g. haloperidol) and agonist ligands (e.g. PRE-084), with an affinity of at least 10%, at least 20%, at least 30%, at least 40%, at least 50%, at least 60%, at least 70%, at least 80%, or at least 90% of the wildtype human σ₁ receptor. Preferably, a functionally equivalent variant of σ₁ receptor has an amino acid sequence identity of at least 70%, at least 80%, at least 90%, at least 95%, at least 98%, or at least 99% to its wildtype counterpart, preferably human σ₁ receptor. In another embodiment, it may differ from its wildtype counterpart, preferably human σ₁ receptor, by up to 30, up to 20, up to 15, up to 10, up to 5, or up to 3 amino acid substitutions, preferably conservative amino acid substitutions, or deletions. Also, it may have up to 50, up to 40, up to 30, up to 20, up to 15, up to 10, up to 5, or up to 3 amino acid insertions.

Also, variants of the σ₁ receptor may be those which contain one or more modified amino acid residues, e.g., residues that are modified by the attachment of substituent groups; those in which the protein is a splice variant σ₁ receptor, e.g. according to NCBI reference numbers NP_005857.1 or NP_671513.1 for human σ₁ receptor; and/or fragments of the proteins. Fragments preferably have a length of at least 60%, at least 70%, at least 80%, at least 90%, at least 95%, or preferably at least 98% of wildtype counterpart, preferably human σ₁ receptor. The fragments include proteins generated via naturally occuring proteolytic cleavage (including multi-site proteolysis) of an original sequence, particularly in the sense of post-translational modifications.

Further, variants can be post-translationally modified. For example, post-translational modifications that fall within the scope of the present invention include signal peptide cleavage, glycosylation, acetylation, isoprenylation, proteolysis, myristoylation, protein folding and proteolytic processing, etc. Additionally, the proteins may include unnatural amino acids formed by post-translational modification or by introducing unnatural amino acids during translation.

In the context of the present invention, the term "FRET" means "Förster Resonance Energy Transfer" or, if fluorescence is employed for both chromophores, "fluorescence resonance energy transfer", and refers to the radiationless transmission of an energy quantum from its site of absorption (the donor) to the site of its utilization (the acceptor) in a molecule, or system of molecules, by resonance interaction between donor and acceptor species, over distances considerably greater than interatomic, without substantial conversion to thermal energy, and without the donor and acceptor coming into kinetic collision. It includes equivalent methods such as BRET (Bioluminescence Resonance Energy Transfer). Also within the scope of the invention are FRET variants wherein the light donated by the donor is not necessarily due to fluorescence, but another kind of light emission, such as lumiscence, for example bioluminescence, chemiluminescence, electrochemiluminescence, electroluminescence, cathodoluminescence, triboluminescence, photoluminescence, phosphorescence, radioluminescence, or thermoluminescence. Also, the light acceptor may be any chromophore.

The term "donor fluorescent protein moiety" as used herein relates to a chemical or biological molecule that initially absorbs energy (e.g., optical energy or electronic energy) and whose emitted energy is absorbed by another molecule called "acceptor". The term "acceptor fluorescent protein moiety" as used herein relates to a chemical or biological molecule that accepts energy via resonance energy transfer. As used herein, such a donor fluorescent moiety and an acceptor fluorescent moiety are referred to "FRET pair".

In FRET, the "donor fluorescent protein moiety" and the "acceptor fluorescent protein moiety" (the "FRET pair") are selected so that the donor and acceptor moieties exhibit FRET when the donor moiety is excited and if they are in close proximity (<20 nm, <15 nm, <12 nm, <10 nm, <8 nm or <5 nm, preferably, <10 nm). One factor to be considered in choosing the donor/acceptor fluorescent protein moiety pair is the efficiency of FRET between the two moieties. Preferably, the efficiency of FRET between the donor and acceptor moieties is at least 10%, preferably at least 30%, more preferably at least 50%, even more preferably at least 70% and most preferably at least 90%. The efficiency of FRET can be tested empirically using the methods known in the art. It also depends on the distance between donor and acceptor, and thus distances can be measured between proteins or protein moieties. For details, see Langois and collaborators (Langois et al. 1976, J. Mol. Biol. 106, 297-313; Langois et al. 1977, Biochemistry 16, 2349-2356). For example, a FRET pair can be chosen based on particular requirements for the protein being studied such as distance between protein components being tested, subcellular location of the protein (example, is the protein in the ER or Golgi, peripheral vesicles or nucleus) and the type of microscopy being used to measure FRET (example, confocal versus TIRF). Criteria for an optimal pair are a large Fluorescence radius, higher quantum yield of the acceptor fluorophore and increased photostability of donor and acceptor fluorophores.

By "fluorescent protein" is meant any protein capable of emitting light when excited with appropriate electromagnetic radiation/light (i.e. light of an appropriate wavelength). The fluorescent protein will absorb energy of a specific wavelength and re-emit energy at a different (but equally specific) wavelength. Fluorescent proteins that can be used include biological and chemical fluorophores. Exemplary biological fluorophores comprise T-sapphire, Cerulean, mCFPm, CyPet, EGFP, PA-EGFP, Emerald, EYFP, Venus, mCitrine, mKO, mOrange, DSRed, JRed, mStrawberry, mCherry, PA-mCherry, mRuby, Tomato, mPlum, mKate, mKatushka, Kaede, Halotag, and superecliptic fluorine. Exemplary chemical fluorophores comprise Alexafluor, Rhodamine, BODIPY, Tetramethylrhodamine, Cyanin dyes, Fluorescein, Quantum dots, IR dyes, FM dyes, ATTO dye. FRET pairs of known fluorophores can be established based on their light excitation and emission profile. Examples include: BFP-GFP; CFP-dsRED; BFP-GFP; Cy3-Cy5; CFP-YFP; Alexa488-Alexa555; Alexa488-Cy3; FITC-TRITC; and DiSBAC⁴(3)-CC2-DMPE.

In a particular embodiment, the donor fluorescent protein moiety and/or the acceptor fluorescent protein moiety are Aequorea-related fluorescent protein. A fluorescent protein is an "Aequorea-related fluorescent protein" if any contiguous sequence of 150 amino acids of the fluorescent protein has at least 85%, at least 90%, or at least 95% sequence identity with an amino acid sequence, either contiguous or non-contiguous, from the wild type Aequorea green fluorescent protein. More preferably, a fluorescent protein is an Aequorea-related fluorescent protein if any contiguous sequence of 200 amino acids of the fluorescent protein has at least 95%, preferably at least 98% sequence identity with an amino acid sequence, either contiguous or non-contiguous, from the wild type Aequorea green fluorescent protein. Similarly, the fluorescent protein can be related to Renilla or Phialidium wild-type fluorescent proteins using the same standards. A variety of Aequorea-related GFPs having useful excitation and emission spectra have been engineered by modifying the amino acid sequence of a naturally occurring GFP from Aequorea Victoria (Prasher et al. 1992, Gene 111, 229-233; Heim et a1.1994, Proc. Natl. Acad. Sci. USA 91, 12501-12504; U.S. Ser. No. 08/337,915, filed Nov. 10, 1994; International application PCT/US95/14692, filed Nov. 10, 1995; and U.S. Ser. No. 08/706,408, filed Aug. 30, 1996). The cDNA of GFP can be fused with those encoding many other proteins; the resulting fusions often are fluorescent and retain the biochemical features of the partner proteins (Cubitt et al. 1995, TiBS 20, 448-455). Mutagenesis studies have produced GFP mutants with shifted wavelengths of excitation or emission (Heim and Tsien 1996, Current Biol. 6, 178-182). Suitable pairs, for example a blue-shifted GFP mutant P4-3 (Y66H/Y 145F) and an improved green mutant S65T can respectively serve as a donor and an acceptor for FRET (Tsien et al. 1993, Trends Cell. Biol. 3, 242-245). Other fluorescent proteins can be used as the fluorescent moiety, such as, for example, yellow fluorescent protein from Vibrio fischeri strain Y-1, Peridinin-chlorophyll a binding protein from the dinoflagellate Symbiodinium sp. phycobiliproteins from marine cyanobacteria such as Synechococcus, e.g., phycoerythrin and phycocyanin, or oat phytochromes from oat reconstructed with phycoerythrobilin. These fluorescent proteins have been largely described (Baldwin et al. 1990, Biochemistry 29, 5509-5515; Morris et al. 1994, Plant Mol. Biol. 24, 673-677; Wilbanks et al. 1993, J Biol. Chem. 268, 1226-1235; Li et al. 1995, Biochemistry 34, 7923-7930).

In a particular embodiment, the donor fluorescent protein moiety is the Cyan Fluorescent Protein (CFP) and/or the acceptor fluorescent protein moiety is the Yellow Fluorescent Protein (YFP). As those skilled in the art will appreciate, the terms CFP and YFP also include mutant forms of said proteins that possess the fluorescence excitation and emission properties similar, respectively, to the CFP and YFP (the latter including second generation and third generation YFP mutants including Citrine and Venus).

In some embodiments, the FRET pair can be an enhanced CFP (ECFP)-YFP (EYFP) pair. Both are color variants of GFP. Each FRET pair possesses unique advantages that may make it more suitable than others for particular proteins of interest. For example, the ECFP-EYFP pair displays a large spectral overlap between donor emission and acceptor excitation, allowing for robust FRET. In addition, EYFP has a high quantum yield and is therefore very suitable as a FRET acceptor. The EGFP-mCherry pair demonstrates a large Fluorescence radius, allowing for FRET measurements in proteins with a large distance separating the fluorophores. The spectral overlap between EGFP emission and mCherry is minimal, thus negating false FRET measurements because of donor crosstalk and bleedthrough. EGFP is highly photostable.

According to the first aspect of the invention, the σ₁ receptor comprised in the fusion protein is flanked by the donor fluorescent protein moiety and the acceptor fluorescent protein moiety. In a preferred embodiment, the donor fluorescent protein moiety is located N-terminally of the σ₁ receptor, and the acceptor fluorescent protein moiety is located C-terminally of the σ₁ receptor. In another embodiment, the acceptor fluorescent protein moiety is located N-terminally of the σ₁ receptor, and the donor fluorescent protein moiety is located C-terminally of the σ₁ receptor.

The use of recombinant DNA techniques to create a fusion gene, with the translational product being the desired test fusion proteins, is well known in the art. Essentially, the joining of various DNA fragments coding for different polypeptide sequences is performed in accordance with conventional techniques, employing blunt-ended or stagger-ended termini for ligation, restriction enzyme digestion to provide for appropriate termini, filling in of cohesive ends as appropriate, alkaline phosphatase treatment to avoid undesirable joining, and enzymatic ligation. Alternatively, the fusion gene can be synthesized by conventional techniques including automated DNA synthesizers. In another method, PCR amplification of gene fragments can be carried out using anchor primers which give rise to complementary overhangs between two consecutive gene fragments which can subsequently be annealed to generate a chimeric gene sequence.

In a further embodiment, the fusion protein of the first aspect comprises a polypeptide linker between the acceptor fluorescent protein moiety and the σ₁ receptor and/or between the donor fluorescent protein moiety and the σ1receptor. Such a linker can facilitate enhanced flexibility of the fusion protein, and it can also reduce steric hindrance between the two fragments, and allow appropriate interaction between the two test polypeptide portions. The linker can also facilitate the appropriate folding of each fragment to occur. The linker can be of natural origin, such as a sequence determined to exist in random coil between two domains of a protein. An exemplary linker sequence is the linker found between the C-terminal and N-terminal domains of the RNA polymerase [alpha] subunit. Other examples of naturally occurring linkers include linkers found in the [lambda]cI and LexA proteins. Alternatively, the linker can be of synthetic origin. For instance, the sequence (Gly4Ser)3 can be used as a synthetic unstructured linker. Linkers of this type are described in Huston et al. 1988, Proc. Natl. Acad. Sci. USA 85, 4879; and U.S. Pat. No. 5,091,513. Another exemplary embodiment includes a poly alanine sequence, e.g., (Ala)3.

The fusion protein of the first aspect unexpectedly has the property that the conformation of the σ₁ receptor changes upon binding of an antagonist such that the donor fluorescent protein moiety and to the acceptor fluorescent protein moiety are brought closer to each other. Therefore, FRET and consequently the light emission of the acceptor fluorescent protein increases upon binding of an antagonist. Accordingly, in one embodiment, the present invention relates to the fusion protein according to the first aspect, wherein energy transfer from the donor fluorescent protein moiety in an excited state to the acceptor fluorescent protein moiety increases if a σ₁ receptor antagonist binds to the σ₁ receptor. In other words, the present invention relates to the fusion protein according to the first aspect, wherein energy transfer from the donor fluorescent protein moiety in an excited state to the acceptor fluorescent protein moiety is increased if a σ₁ receptor antagonist is bound to the σ₁ receptor compared to the energy transfer from the donor fluorescent protein moiety in an excited state to the acceptor fluorescent protein moiety wherein no ligand is bound to said fusion protein.

Even more surprisingly, the present inventors have found that the conformation of the σ₁ receptor changes upon binding of an agonist such that the donor fluorescent protein moiety and to the acceptor fluorescent protein moiety are removed further from each other. Therefore, FRET and consequently the light emission of the acceptor fluorescent protein decreases upon binding of an agonist. Accordingly, in another embodiment, the present invention relates to the fusion protein according to the first aspect, wherein energy transfer from the donor fluorescent protein moiety in an excited state to the acceptor fluorescent protein moiety decreases if a σ₁ receptor agonist binds to the σ₁ receptor. In other words, the present invention relates to the fusion protein according to the first aspect, wherein energy transfer from the donor fluorescent protein moiety in an excited state to the acceptor fluorescent protein moiety is decreased if a σ₁ receptor agonist is bound to the σ₁ receptor compared to the energy transfer from the donor fluorescent protein moiety in an excited state to the acceptor fluorescent protein moiety wherein no ligand is bound to said fusion protein.

The present inventors have designed the fusion protein of the invention such that the donor fluorescent protein moiety and the acceptor fluorescent protein are in close proximity (<10 nm) to each other, i.e. there is light emission of the acceptor fluorescent protein, if no ligand is bound to the σ₁ receptor. Accordingly, in another embodiment, the present invention relates to the fusion protein according to the first aspect, wherein energy transfer from the donor fluorescent protein moiety in an excited state to the acceptor fluorescent protein moiety occurs if no ligand is bound to the σ₁ receptor.

This has the unforeseeable advantage that light emission of the acceptor fluorescent protein can indicate and distinguish between three states: "not bound to a ligand", "bound to a ligand which is an antagonist" and "bound to a ligand which is an agonist". Thus, in preferred embodiment, the present invention relates to the fusion protein according to the first aspect, wherein energy transfer from the donor fluorescent protein moiety in an excited state to the acceptor fluorescent protein moiety occurs if no ligand is bound to the σ₁ receptor, wherein energy transfer from the donor fluorescent protein moiety in an excited state to the acceptor fluorescent protein moiety increases if a σ₁ receptor antagonist binds to the σ₁ receptor, and wherein energy transfer from the donor fluorescent protein moiety in an excited state to the acceptor fluorescent protein moiety decreases if a σ₁, receptor agonist binds to the σ₁ receptor.

In a further embodiment, the invention relates to the fusion protein according to the first aspect, further comprising an N-terminal or C-terminal localization signal peptide.

The term "localization signal peptide" refers to a short (3-60 amino acids long) peptide chain that directs the transport of a protein. It is also known in the art as targeting signal, signal sequence, transit peptide, or localization signal or equivalent terms. The amino acid sequences of localization signal peptides direct proteins (which are synthesized in the cytosol) to certain cellular locations or organelles such as the cell membrane, the nucleus, mitochondrial matrix, endoplasmic reticulum, chloroplast, apoplast or peroxisome. Some signal peptides are cleaved from the protein by a signal peptidase after the proteins are transported.

Different localization signal peptides allow studying the function of the σ₁ receptor in different primary cellular locations. This is potentially beneficial for identifying ligands which occur only or primarily in particular cellular locations. For example, in a cell-based ligand screening assay, wherein potential ligands are added to the cell medium, a localization signal peptide directing the fusion protein of the invention to the plasma membrane can increase the sensitivity if the assay, since all or at least a higher proportion of the fusion proteins expressed by the cells will be located at the plasma membrane, where they are more exposed to the ligands, rather than intracellularly.

Accordingly, in a preferred embodiment, said localization signal peptide directs the fusion protein of the first aspect to the plasma membrane. Also, other signal peptides may be comprised in the fusion protein of the first aspect to redirect and/or retain it to/in a certain organelle (e.g. nucleus, mitochondria, endoplasmic reticulum or peroxisome). Non-limiting examples for localization peptides (with corresponding exemplary amino acid sequences) are: Nuclear localization signal (e.g. PPKKKRKV, SEQ ID NO: 8); Transport to the mitochondrial matrix signal (e.g. H₂N-MLSLRQSIRFFKPATRTLCSSRYLL-, SEQ ID NO: 9); Transport to the endoplasmic reticulum signal (e.g. H₂N-MMSFVSLLL VGILFW ATEAEQLTKCEVFQ-, SEQ ID NO: 10); Endoplasmic reticulum retention signal (e.g. -KDEL-COOH, SEQ ID NO: 11); Peroxisomal targeting signal (e.g. -SKL-COOH). The H₂N is the N-terminus of the fusion protein and COOH is the C-Terminus of the fusion protein including the localization signal peptide.

Preferably, said plasma membrane signal peptide is selected from the group consisting of human PTH1R signal peptide (H₂N-MGTARIAPGLALLLCCPVLSSAYAL-, SEQ ID NO: 5), human mGluR₅ signal peptide (H₂N-MVLLLILSVLLLKEDVRGSA-, SEQ ID NO: 6) and human GABA_{B2}R signal peptide (H₂N-MASPRSSGQPGPPPPPPPPPARLLLLLLLP LLLPLAPG-, SEQ ID NO: 7).

While it is apparent to the skilled person that modifications and variations of the fusion protein, for example as set out above, can be made within the scope of the invention, in a most preferred embodiment, the invention relates to the fusion protein according to the first aspect, wherein said fusion protein has the amino acid sequence according to SEQ ID NO: 1 or SEQ ID NO: 2.

In another embodiment, the fusion protein of the first aspect may be comprised in a membrane. Said membrane preferably is a phospholipid bilayer. It may derived from a prokaryotic or eukaryotic cell or it may be formed *in vitro,* e.g. as a liposome.

Further, the present invention relates to the use of the fusion protein and/or the membrane of the third aspect for identifying a ligand for a σ₁ receptor. In a preferred embodiment, said ligand is an antagonist. In another preferred embodiment, said ligand is an agonist.

### POLYNUCLEOTIDES OF THE INVENTION

In a second aspect, the invention relates to a polynucleotide comprising a coding sequence encoding a fusion protein according to the first aspect.

In one embodiment, said polynucleotide is a coding sequence encoding said fusion protein, such as an mRNA or a cDNA. Preferably, said coding sequence has the sequence according to SEQ ID NO: 3 or SEQ ID NO: 4 or a variant sequence at least 70 %, at least 80 %, at least 90 %, at least 95 %, or at least 99 % identical thereto, wherein said variant sequence encodes for a variant protein having the functions of the fusion protein of the invention or its components as described herein, e.g. that of the σ₁ receptor as described above and that of a donor and an acceptor fluorescent protein moiety.

In another embodiment, the polynucleotide of the second aspect can be an expression cassette comprising said coding sequence. The term "expression cassette" refers to a DNA sequence comprising a regulator, preferably promoter, sequence, an open reading frame, and a 3' untranslated region, which preferably is, in eukaryotes, a polyadenylation site. The expression cassette can be part of vector used for cloning and transformation. Different expression cassettes can be transformed into different organisms including bacteria, yeast, plants, and mammalian cells as long as appropriate regulatory sequences, i.e. promoters, are used. Thus, said polynucleotide may also be a vector comprising said coding polynucleotide. Vectors, such as plasmids (e.g. the pEYFP-N1 vector (Clontech, Mountain View, CA, USA)), viruses, cosmids etc., as well as promoters, e.g. the human cytomegalovirus (hCMV) promoter, that can be used in the present invention are well known in the art.

Polynucleotides, expression cassettes and vectors of the invention can be obtained by means of the use of well known techniques in the state of the art (as described, e.g., in Sambrook et al., (2001), "Molecular cloning, a Laboratory Manual", 3rd ed., Cold Spring Harbor Laboratory Press, N.Y., Vol 1-3).

Further, the present invention relates to_the use of the polynucleotide of the second aspect for identifying a ligand for a σ₁ receptor. In a preferred embodiment, said ligand is an antagonist. In another preferred embodiment, said ligand is an agonist.

### CELL OF THE INVENTION

In a third aspect, the invention relates to a cell comprising the fusion protein of the first aspect, the membrane comprising the fusion protein of the first aspect and/or the polynucleotide of the second aspect.

Cells to be used can be any cell types, including both eukaryotic cells and prokaryotic cells. Preferably, they can be engineered to contain fluorescent moieties suitable for the assay. More preferably, the cells include prokaryotic cells, yeast cells, or mammalian cells. Preferred examples of mammalian cells are for instance HEK-293 cells, MOLT-3 cells, COS cells, HeLa cells, and also cells of established human cancer cell lines, cells derived/isolated from the central nervous system (CNS), particularly from the CNS of patients suffering from psychosis and movement disorders such as dystonia or tardive dyskinesia, and/or motor disturbances associated with Huntington's chorea, Tourette's syndrome or Parkinson's disease, etc. In a particular embodiment, the host cell is a HEK (human embryonic kidney) or HEK-293 cell. In addition, cells should preferably be able to express the fusion protein of the invention in a soluble and functional state.

Further, the present invention relates to_the use of the cell of the third aspect for identifying a ligand for a σ₁ receptor. In a preferred embodiment, said ligand is an antagonist. In another preferred embodiment, said ligand is an agonist.

### METHOD OF THE INVENTION

As set out above, the effort of the inventors aimed at providing an assay for identifying ligands of the σ₁ receptor. The essential tool which was developed for this is the fusion protein of the first aspect. Based on this fusion protein, the inventors have devised a method for identifying ligands of the σ₁ receptor, which takes advantage of both the positions of the fluorophores when no ligand is bound to the receptor and the change in their position upon binding of a ligand.

Accordingly, in a fourth aspect, the present invention relates to a method for identifying a ligand for a σ₁ receptor comprising:
(i) exposing a fusion protein according to the first aspect, a fusion protein comprised in a membrane according to the first aspect, or a fusion protein comprised in a cell according to third aspect to light of a wavelength exciting the donor fluorescent protein moiety, upon which the donor fluorescent protein moiety fluoresces at a first emission wavelength, wherein the light of the first emission length is capable of exciting the acceptor fluorescent protein moiety;
(ii) detecting light of a second emission wavelength emitted by the acceptor protein moiety;
(iii) contacting the fusion protein, membrane and/or cell of step (i) with a test compound; and
(iv) detecting light of a second emission wavelength emitted by the acceptor protein moiety of the fusion protein of step (iii) and, optionally, a control;
wherein, if the intensity of light of a second emission wavelength emitted during step (i) is known or if a control is included, step (ii) may be omitted and, if omitted, step (iii) may be carried out before step (i), and wherein the test compound is identified as a ligand for a σ₁ receptor if there is a difference in the intensity of light of the second emission wavelength detected in step (iv) and
- the intensity of light of the second emission wavelength detected in step (ii),
- the intensity of light of the second emission wavelength known to be emitted during step (i), or
- the intensity of light of the second emission wavelength of the control.

In other words, the method of the fourth aspect may be formulated alternatively as a method for identifying a ligand for a σ₁ receptor comprising:
(i) contacting a fusion protein according to the first aspect, a fusion protein comprised in a membrane according to the first aspect, or a fusion protein comprised in a cell according to the third aspect with a test compound; and
(ii) detecting light of a second emission wavelength emitted by the acceptor protein moiety of the fusion protein of step (i) and, optionally, of a control, upon which the donor fluorescent protein moiety fluoresces at a first emission wavelength, wherein the light of the first emission length is capable of exciting the acceptor fluorescent protein moiety;
wherein said fusion protein is exposed to light of a wavelength exciting the donor fluorescent protein moiety before step (i) and/or between step (i) and step (ii), and wherein the test compound is identified as a ligand for a σ₁ receptor if there is a difference in the intensity of light of the second emission wavelength detected in step (ii) and
- the intensity of light of the second emission wavelength known to be emitted before step (i), or
- the intensity of light of the second emission wavelength of the control.

Preferably, therein the intensity of light of the second emission wavelength known to be emitted before step (i) is measured by detecting light of said second emission wavelength emitted by the acceptor protein moiety after said fusion protein is exposed to light of a wavelength exciting the donor fluorescent protein moiety before step (i) or is a predetermined value of the intensity of light of the second emission wavelength emitted by said fusion protein when not bound to a ligand and when exposed to light of a wavelength exciting the donor fluorescent protein moiety.

As yet another alternative, the method of the fourth aspect may also be described as a method or collection of methods encompassing one or more of the following five variations (A)-(E), which all achieve the same goal:
(A) A method for identifying a ligand for a σ₁ receptor comprising:
   (i) exposing a fusion protein according to the first aspect, a fusion protein comprised in a membrane according to the first aspect, or a fusion protein comprised in a cell according to the third aspect to light of a wavelength exciting the donor fluorescent protein moiety, upon which the donor fluorescent protein moiety fluoresces at a first emission wavelength, wherein the light of the first emission length is capable of exciting the acceptor fluorescent protein moiety;
   (ii) detecting light of a second emission wavelength emitted by the acceptor protein moiety of said fusion protein;
   (iii) contacting said fusion protein with a test compound; and
   (iv) detecting light of a second emission wavelength emitted by the acceptor protein moiety of said fusion protein;
   wherein the test compound is identified as a ligand for a σ₁ receptor if there is a difference in the intensity of light of the second emission wavelength detected in step (iv) and the intensity of light of the second emission wavelength detected in step (ii).

In a preferred embodiment of variation (A), the exposure in step (i) is maintained throughout steps (i) to (iv). In another preferred embodiment, light is not only detected in steps (ii) and steps (iv), but continuously, i.e. during steps (ii) to (iv).
(B) A method for identifying a ligand for a σ₁ receptor comprising:
   (i) contacting a fusion protein according to the first aspect, a fusion protein comprised in a membrane according to the first aspect, or a fusion protein comprised in a cell according to the third aspect with a test compound;
   (ii) exposing said fusion protein and a control to light of a wavelength exciting the donor fluorescent protein moiety of said fusion protein and of said control, upon which the donor fluorescent protein moiety fluoresces at a first emission wavelength, wherein the light of the first emission length is capable of exciting the acceptor fluorescent protein moiety; and
   (iii) detecting light of a second emission wavelength emitted by the acceptor protein moiety of said fusion protein and of said control;
      wherein the test compound is identified as a ligand for a σ₁ receptor if there is a difference in the intensity of light of the second emission wavelength emitted by the acceptor protein moiety of said fusion protein and the intensity of light of the second emission wavelength emitted by the acceptor protein moiety of the control.

In a preferred embodiment of variation (B), the exposure in step (ii) is maintained throughout steps (ii) to (iii).
(C) A method for identifying a ligand for a σ₁ receptor comprising:
   (i) exposing a fusion protein according to the first aspect, a fusion protein comprised in a membrane according to the first aspect, or a fusion protein comprised in a cell according to the third aspect and a control to light of a wavelength exciting the donor fluorescent protein moiety of said fusion protein and of said control, upon which the donor fluorescent protein moiety fluoresces at a first emission wavelength, wherein the light of the first emission length is capable of exciting the acceptor fluorescent protein moiety;
   (ii) contacting said fusion protein with a test compound; and
   (iii) detecting light of a second emission wavelength emitted by the acceptor protein moiety of said fusion protein and of said control;
   wherein the test compound is identified as a ligand for a σ₁ receptor if there is a difference in the intensity of light of the second emission wavelength emitted by the acceptor protein moiety of said fusion protein and the intensity of light of the second emission wavelength emitted by the acceptor protein moiety of the control.

In a preferred embodiment of variation (C), the exposure in step (i) is maintained throughout steps (i) to (iii).
(D) A method for identifying a ligand for a σ₁ receptor comprising:
   (i) exposing a fusion protein according to the first aspect, a fusion protein comprised in a membrane according to the first aspect, or a fusion protein comprised in a cell according to the third aspect to light of a wavelength exciting the donor fluorescent protein moiety, upon which the donor fluorescent protein moiety fluoresces at a first emission wavelength, wherein the light of the first emission length is capable of exciting the acceptor fluorescent protein moiety;
   (ii) contacting said fusion protein with a test compound; and
   (iii) detecting light of a second emission wavelength emitted by the acceptor protein moiety of said fusion protein;
   wherein the test compound is identified as a ligand for a σ₁ receptor if there is a difference in the intensity of light of the second emission wavelength detected in step (iii) and the intensity of light of the second emission wavelength known to be emitted during step (i).

In a preferred embodiment of variation (D), the exposure in step (i) is maintained throughout steps (i) to (iii).
(E) A method for identifying a ligand for a σ₁ receptor comprising:
   (i) contacting a fusion protein according to any one of the first aspect, a fusion protein comprised in a membrane according to the first aspect, or a fusion protein comprised in a cell according to the third aspect with a test compound;
   (ii) exposing said fusion protein to light of a wavelength exciting the donor fluorescent protein moiety of said fusion protein, upon which the donor fluorescent protein moiety fluoresces at a first emission wavelength, wherein the light of the first emission length is capable of exciting the acceptor fluorescent protein moiety; and
   (iii) detecting light of a second emission wavelength emitted by the acceptor protein moiety of said fusion protein;
   wherein the test compound is identified as a ligand for a σ₁ receptor if there is a difference in the intensity of light of the second emission wavelength detected in step (iii) and the intensity of light of the second emission wavelength known to be emitted by said fusion protein when not bound to a ligand and when exposed to light of a wavelength exciting the donor fluorescent protein moiety.

In a preferred embodiment of variation (E), the exposure in step (ii) is maintained throughout steps (ii) to (iii).

Any method features defined, described, and explained in the following refer to the method of the fourth aspect as described first. However, it is to be understood that these definitions, descriptions or explanations apply equivalently - in as far as they are applicable - to the alternative formulation of the method of the fourth aspect as well as to the method variations (A) to (E). Although it should go without saying, as the step designations (i), (ii), (iii) and/or (iv) do not always correspond between the differently defined methods, this remark applies to the method features and steps as defined by their subject-matter and not necessarily by the step designation.

Preferably, said method of the fourth aspect is an *in vitro* method for identifying a ligand for a σ₁ receptor.

Herein, the term "control" refers inter alia to a negative control, i.e. to a fusion protein according to the first aspect, a membrane according to the first aspect, or a cell according to the third aspect not contacted with a test compound method but otherwise treated according to above method (e.g., the negative control is also exposed to light and emitted light is also detected). In particular, the negative control can be used to identify any ligand, i.e. both agonists and antagonists. The term "control" also includes a positive control, i.e. a fusion protein according to the first aspect, a membrane according to the first aspect, or a cell according to the third aspect contacted with a known ligand (agonist and/or antagonist). In particular, a positive control can be used to identify specifically agonists (if an agonist is used as a positive control) or antagonists (if an antagonist is used as a positive control). It also envisaged the term "control" includes both a negative and a positive control (optionally both agonist and antagonist), i.e. several controls may be used in parallel, e.g. a negative control and a positive control (agonist), a negative control and a positive control (antagonist), or a negative control and two positive controls (agonist and antagonist). When it is referred herein to light emitted by the control, the light emission of the respective fluorescent protein moiety is meant (i.e. acceptor or donor).

As will be understood by the person skilled in the art, step (i) of said method does not necessarily imply that the exposure with light is terminated after step (i) or does not occur during the following steps. As such, step (i) may also be: "(i) initiating the exposure of a fusion protein according to the first aspect, a membrane comprising a fusion protein according to the first aspect, or a cell according the third aspect with light of a wavelength exciting the donor fluorescent protein moiety, upon which, preferably, the donor fluorescent protein moiety fluoresces at a first emission wavelength exciting the acceptor fluorescent protein moiety." In case the exposure with light is terminated after step (i), above method optionally comprises an additional step during step (iii) or between steps (iii) and (iv) of exposing the fusion protein, the membrane, or the cell of step (i) to light of a wavelength exciting the donor fluorescent protein moiety, upon which, preferably, the donor fluorescent protein moiety fluoresces at a first emission wavelength exciting the acceptor fluorescent protein moiety.

In another embodiment, the intensity of light of the first emission wavelength emitted by the donor fluorescent protein moiety may also be detected when that of the second emission wavelength emitted by the acceptor fluorescent protein moiety is detected and a ratio of these values may be calculated, preferably the ratio "intensity of light the second emission wavelength emitted by the acceptor fluorescent protein moiety" divided by the "intensity of light of the first emission wavelength emitted by the donor fluorescent protein moiety".

As set out above, the fusion protein of the first aspect unexpectedly has the property that the conformation of the σ₁ receptor changes upon binding of an antagonist such that the light emission of the acceptor fluorescent protein increases upon binding of an antagonist. Accordingly, in one embodiment, the present invention relates to method according to the fourth aspect, wherein the test compound is identified as an antagonist for a σ₁ receptor if the intensity of light of the second emission wavelength detected in step (iv) is increased compared to the light of the second emission wavelength detected in step (ii), known to be emitted during step (i), or that of the control.

Further, the present inventors have designed the fusion protein of the invention such that there is light emission of the acceptor fluorescent protein if no ligand is bound to the σ₁ receptor. Accordingly, in another embodiment, the present invention relates to the method according to the fourth aspect, wherein the test compound is determined not to be a ligand of a σ₁ receptor if the intensity of light of the second emission wavelength detected in step (iv) does not change compared to the light of the second emission wavelength detected in step (ii), known to be emitted during step (i), or that of the control.

In addition, they have found that the conformation of the σ₁ receptor changes upon binding of an agonist such that the light emission of the acceptor fluorescent protein decreases upon binding of an agonist. Accordingly, in another embodiment, the present invention relates to the method according to the fourth aspect, wherein the test compound is identified as an agonist for a σ₁ receptor if the intensity of light of the second emission wavelength detected in step (iv) is decreased compared to the light of the second emission wavelength detected in step (ii), known to be emitted during step (i), or that of the control.

This has the advantage that the method of the invention can distinguish between three types of test compounds: "no ligand", "antagonist" and "agonist". Thus, in preferred embodiment, the present invention relates to the method according to the fourth aspect, wherein the test compound is identified as an antagonist for a σ₁ receptor if the intensity of light of the second emission wavelength detected in step (iv) is increased compared to the light of the second emission wavelength detected in step (ii), known to be emitted during step (i), or that of the control, wherein the test compound is identified as an agonist for a σ₁ receptor if the intensity of light of the second emission wavelength detected in step (iv) is decreased compared to the light of the second emission wavelength detected in step (ii), known to be emitted during step (i), or that of the control, and wherein the test compound is determined not to be a ligand of a σ₁ receptor if the intensity of light of the second emission wavelength detected in step (iv) does not change compared to the light of the second emission wavelength detected in step (ii), known to be emitted during step (i), or that of the control.

In step (i) of above method, the fusion protein is exposed to light of a wavelength exciting the donor fluorescent protein moiety to potentially induce FRET with respect to the acceptor fluorescent protein moiety. This can be one-photon, two-photon, or multiple photons FRET, depending on the purpose, e.g. reducing the background autofluorescence of cells.

The excitation light source or microscope should be compatible for the purpose of performing the method of the invention for example for assays on live cells. For example, it should be able to overcome the tremendous amounts of light scattering, and thus artifacts, generated by live cells. Preferably, it is an instrument coupling a confocal microscope with a spectrofluorimeter. In one embodiment, the cell of step (i) is a single cell.

It should be understood, though, that confocal laser source is preferably used for excitation of single cells, but many other laser sources may also be applicable under certain conditions, such as when populations of cells rather than single cells are used under low background conditions. It should also be understood that the wavelengths mentioned in this application are for illustrative purpose only, and are by no means limiting. With the discovery of future fluorescent molecules/proteins with unique excitation and emission wavelengths, these wavelengths can also be properly used to practice the instant invention.

In steps (ii) and (iv), the method of the invention comprises detecting emission of the second emission wavelength, and optionally, light of the first emission wavelength emitted by the donor fluorescent protein moiety (e.g. for calculating ratios).

The method of the invention comprises detecting the interaction between donor and acceptor by recording emission intensities and quantifying, e.g. FRET efficiency. The FRET efficiency E is defined by the Fluorescence equation described in Lakowicz, 2006 (Energy Transfers. Principles of Fluorescence Spectroscopy. J.R. Lakowicz, editor. Springer Science, New York, 443-476). In several embodiments, methods of the present disclosure use two classes of techniques that provide approximate E values based on microscope images acceptor photobleach FRET and sensitized FRET (Wallrabe and Periasamy 2005, Curr. Opin. Biotecnol. 16, 19-27; Jares-Erijman and Jovin 2006, Curr. Opin. Chem. Biol. 10, 409-416).

The interaction between donor and acceptor can also be reported by quantification of FRET amplitude as described in the art. The FRET amplitude measured from sensitized emission of the acceptor during excitation by the donor and is not a direct measurement of the FRET efficiency E.

The term FRET amplitude can also be applied to describe the magnitude of any FRET signal calculated by any known method to quantify FRET from sensitized emission images that is not a direct measure of the FRET efficiency E. The acquired FRET amplitude is an indirect measure of E (Elangovan et al. 2003, Methods 29, 58-73.) and responds nonlinearly to variations in the extent of interaction between fluorophoretagged molecules (Gordon et al. 1998, Biophys. J. 74, 2702-2713).

The FRET imaging acquisition can also be performed by acceptor photobleaching. In a specimen expressing both donor- and acceptor-tagged molecules, the existence of FRET causes a decrease in the donor intensity, proportional to the number of donor-tagged molecules that interact with acceptor-tagged molecules. Thus, "acceptor photobleach" FRET directly measures the FRET efficiency E by quantifying the increase in the donor intensity following photobleach of the acceptor (although artifacts including acceptor photoconversion and donor photobleach can distort this measurement (Rizzo et al. 2006, Microsc. Microanal. 12, 238-254).

The FRET imaging acquisition can also be performed by sensitized emission FRET. The acceptor displays sensitized emission during excitation of the donor. Measurements of such "sensitized FRET" or "sensitized emission FRET" preserves the fluorophores in the sample. Rather than a acquiring a time series of images, it requires the acquisition of either a single image by a device capable of detecting the entire emission spectrum of both the donor and acceptor fluorescence which is then spectrally resolved through linear unmixing of the full-spectrum image into donor, acceptor and FRET images or alternatively using a standard wide-field or confocal fluorescence imaging microscope, the use of three different fluorescence filter cubes appropriate for acquiring images of: 1), the donor channel (IDD, donor excitation and emission), 2), the FRET channel (IDA, donor excitation, acceptor emission), and 3), the acceptor channel (IAA, acceptor excitation and emission).

In a preferred embodiment, light is detected and quantified, in other words FRET is monitored, as the emission ratio between the acceptor emission intensity (F_{acceptor-emission-wavelenght}) and the donor emission intensity (F_{donor-emission-wavelenght}) after excitation of the donor with the corresponding wavelenght. The emission intensity ratio (F_{acceptor-emission-}wavelenght/F_{donor-emission-wavelenght}) is corrected by the respective spillover, namely the donor emission into the acceptor emission wavelength channel and the spillover of the acceptor emission into the donor emission wavelength channel, thus giving the corrected FRET ratio (F*_{acceptor}-ₑₘᵢₛₛᵢₒₙ-_{wavelenght}/F*_{donor-enission-wavelenght}) (see Vilardaga et al. 2003, Nature Biotechnology 21, 807-812).

The term "contacting" refers to bringing two substances into contact with each other and comprises adding the second substance to an experimental setting already comprising the first substance or adding the first substance to an experimental setting already comprising the second substance. Accordingly, the test compound may be added to the fusion protein already in the experimental setting or the fusion protein may be added to the test compound already in the experimental setting. In a preferred embodiment, a cell or cells of the invention are contacted or perfused with the test compound.

In a preferred embodiment, said test compound is an inorganic compound, an organic compound, and/or a biological compound. Examples for inorganic compounds are metals, non-metals, oxides, bases, salts and acids. Organic compounds can be, for example, small organic compounds or polymers. Biological compounds are compounds synthesized by living organisms, preferably those containing carbon atoms as well as nitrogen, sulfur, phosphorus and/or oxygen atoms. They include, e.g., amino acids, peptides, proteins, nucleic acids, polynucleotides, carbohydrates and lipids. In a preferred embodiment, the compounds tested using the method of the invention are from compound libraries, such as small compound libraries, peptide libraries and the like, for example for high-throughput screening. Such libraries are widely used and are commercially available.

The method herein described can be performed with the aids of a computer-readable medium that contains a set of instructions that causes a computer to perform at least one of the methods herein described. Exemplary software is described in Feige et al, 2005, Microsc. Res. Tech. 68, 51-58. Additional exemplary software is provided by written ImageJ macros that can be used to enable assembly of the image stacks ready for analysis and Matlab macros that compile all the pixels from each dataset into a single FRET distribution.

A computer-readable medium can also be included in a computer. In some embodiments, the computer can be the same machine included in the confocal imaging system that acquired the data to allow immediate processing of the newly acquired data.

### KIT OF THE INVENTION

Additionally, the present invention relates to a kit useful to put into practice the invention disclosed herein.

Thus, in another aspect, the invention relates to a kit, hereinafter kit of the invention, comprising the fusion protein, the polynucleotide, the expression cassette, the vector, the cell of the invention, and/or reagents for carrying out the methods of the invention.

In the present invention a "kit" is understood as a product containing the different agents and material to identify a ligand for a σ₁ receptor according to the method of the invention. Illustrative examples of agents useful to identify a ligand for a σ₁ receptor are medium to keep cells, buffers, saline, coverslips, etc. On the other hand, the kit can include a well plate comprising the cells of the invention.

Another component which can be present in the kit is a packing which allows maintaining the agents properly stored. Suitable materials for preparing such packagings include glass, plastic (polyethylene, polypropylene, polycarbonate and the like), bottles, vials, paper, sachets and the like. The kit of the invention can additionally contain instructions for using the agents in the method of the invention. Said instructions can be found in the form of printed material or in the form of an electronic support which can store instructions such that they can be read by a subject, such as electronic storage media (magnetic disks, tapes and the like), optical media (CD-ROM, DVD) and the like. The media can additionally or alternatively contain internet websites providing said instructions.

### EXAMPLES

The following examples are to be construed as illustrative and not limitative of the scope of the invention.

### Materials and Methods

### Plasmid constructs

The constructs presented here were made using standard molecular biology techniques employing PCR and fragment replacement strategy. Thus, two σ₁ receptor FRET sensor constructs were obtained using the cDNA encoding the human σ₁ receptor gene. To this end, the σ₁ receptor was first amplified using the sense oligonucleotide primer (FSEcoRI: 5'-CGGAATTCATGCAGTGGGCCG-3' [SEQ ID NO: 12]) and the antisense primer (RSBamHI: 5'-CAGGATCCCGAGGGTCCTGGCCAAAGAG-3' [SEQ ID NO: 13]). The fragment was then subcloned in-frame into *Eco*RI/*Bam*HIsites of the pEYFP-N1 vector (Clontech, Mountain View, CA, USA), thus resulting in the σ₁ receptor containing the yellow fluorescent protein (YFP) at its C-terminal tail (σ₁^{YFP} construct). Next, to obtain the first σ₁ receptor FRET sensor, the CFP from the PTHR^{CFP} construct (kindly provided by JP Vilardaga, University of Pittsburgh, USA) was amplified using the sense primer (FCFPHindIII: 5'-CGAAGTTCatggtgagcaagggcgaggagc-3' [SEQ ID NO: 14]) and the antisense primer (RCFPEcoRI: 5'-ccGGAATTCcttgtacagctcgtccatgcc-3' [SEQ ID NO: 15]). The fragment encoding the CFP protein was then subcloned upstream in-frame into *Hind*III/*Eco*RI sites of the σ₁^{YFP}-containing pEYFP-N1 plasmid, thus resulting in the σ₁^{CFP/YFP} construct. Finally, to obtain the second σ₁ receptor FRET sensor containing the signal peptide of the PTHR, the CFP from the PTHR^{CFP} construct was amplified using the sense primer (FspCFPHindIII: 5'-GTTTAAACTTAAGTTCGG-3' [SEQ ID NO: 16]) and the antisense primer RCFPEcoRI [SEQ ID NO: 8]. The fragment encoding the PTHR signal peptide together with the CFP protein in frame was then subcloned upstream in-frame into *Hind*III/*Eco*RI sites of the σ₁^{YFP}-containing pEYFP-N1 plasmid, thus resulting in the σ₁^{spCFP/YFP} construct. All constructs were verified by nucleotide sequencing.

### Cell culture and transfection

Human embryonic kidney (HEK)-293T cells were grown at 37°C in an atmosphere of 5% CO₂ in Dulbecco's modified Eagle's medium (Sigma-Aldrich, St. Louis, MO, USA) supplemented with 1 mM sodium pyruvate, 2 mM 1-glutamine, 100 U/mL streptomycin, 100 mg/mL penicillin and 5% (v/v) fetal bovine serum. The cells were seeded into six-well plates containing poly-D-lysine-coated coverslips (18 mm in diameter) with approximately 300,000 cells/well. Cells were transiently transfected with the corresponding cDNA constructs using Transfectin (Bio-Rad, Hercules, CA, USA) and following the manufacturer's instructions. Two stably transfected HEK-293 cell lines expressing the σ₁^{CFP/YFP} and the σ₁^{spCFP/YFP} constructs were obtaining after geneticin (G418) resistance selection (200 µg/ml).

### Single-cell real-time intamolecular-FRET (iFRET)

In the single cell real time intramolecular-FRET approach both donor (i.e. CFP) and acceptor (i.e. YFP) are located in the same molecule, as designed for the σ₁^{CFP/YFP} and the σ₁^{spCFP/YFP} constructs (Figure 1A). In brief, HEK cells permanently expressing the corresponding σ₁ receptor FRET sensor were seeded in poly-D-Lysine coated coverslips and allow growing overnight. Then, the coverslips with transfected cells were mounted in an Attofluor holder and placed on an inverted Axio Observer microscope (Zeiss, Jena, Germany) equipped with a 63X oil immersion objective and a dual-emission photometry system (Till Photonics, Gräfelfing, Germany). The extracellular buffer was Hank's Balanced Salt Solution (HBSS), containing (in mM): 137 NaCl, 0.34 Na2HPO4, 5 KCI, 0.44 KH2PO4, 0.5 MgCl2, 0.4 Mg2SO4, 1.26 CaCl2, 10 HEPES, 2 D-glucose and 1 ascorbic acid (pH 7.4 with NaOH).

A Polychrome V (Till Photonics) was used as the light source. The sample was illuminated with excitation light at 436 ± 10 nm (beam splitter dichroic long-pass; DCLP 460 nm). Excitation time was 10 ms at 10 Hz, in order to limit photobleaching. Emission light intensities were determined at 535 ± 15 and 480 ± 20 nm (DCLP 505 nm). No corrections for spillover between channels or direct YFP excitation were made. Single cells were selected for recording based on their σ₁ expression as judged by its fluorescence. In control experiments (in the absence of agonist application) photobleaching of YFP and CFP followed a time course which was well-described by a monoexponential function. The ligands were applied using a pressure-driven, computer-controlled perfusion system (Octaflow; ALA Scientific Instruments, Westbury, NY, USA) and experiments were carried out at room temperature. Finally, a Digidata 1440 analog/digital converter (Molecular Devices, Sunnyvale, CA, USA) was used for interfacing the photometry system and the perfusion system with a personal computer, which was used to control these systems and to record data. Finally, pCLAMP (Molecular Devices) and GraphPad Prism (GraphPad Software Inc., San Diego, CA, USA) software were used for data collection and analysis.

Eventually, the FRET efficiency between donor (i.e. CFP) and acceptor (i.e. YFP) fluorophores was determined by donor recovery after acceptor photobleaching, in which if FRET occurs then the bleaching of the acceptor yields a significant increase in fluorescence of the donor. In brief, cells expressing the σ₁^{spCFP/YFP} construct were mounted and observed, and the emission light intensities were determined at 535 nm (YFPₚᵣₑ) and 480 nm (CFPₚᵣₑ) as described above. No corrections for spillover between channels or direct YFP excitation were made. Subsequently, acceptor photobleaching was performed by direct illumination of YFP at 500 nm for 5 min. Finally, the emission intensities of YFP and CFP were recorded again (YFPₚₒₛₜ and CFPₚₒₛₜ, respectively). FRET efficiency was calculated according to the equation: FRET efficiency = 1 - (CFPₚᵣₑ/CFPₚₒₛₜ).

### Results

Two σ₁ receptor constructs were generated carrying CFP and YFP in the N and C terminus of the σ₁ receptor, respectively (i.e. σ₁^{CFP/YFP} and σ₁^{spCFP/YFP} (**Figure 1A**)_{.} These two σ₁ receptor constructs were biochemically characterized and their cell surface expression analyzed. Apparently, both constructs expressed well when transiently transfected in HEK-392T cells, thus the presence or absence of the PTHR signal peptide did not affect the total protein expression levels of both constructs. However, when the subcellular distribution of these two constructs was analyzed by means of confocal microscopy, it was clear that the construct carrying the PTHR signal peptide (σ₁^{spCFP/YFP}) showed a preferential plasma membrane distribution when compared to the σ₁^{spCFP/YFP} that accumulated intracellularly **(****Figure 1B****).** To further confirm these differences in cell surface expression, biotinylation experiments were performed to determine their relative plasma membrane expression **(****Figure 1C****).** Interestingly, these experiments showed that σ₁^{spCFP/YFP} was expressed around four-fold more in the plasma membrane when compared with the σ₁^{CFP/YFP} construct (P<0.05) **(****Figure 1C****).** Similar results were obtained with the σ₁^{CFP/YFP} and the σ₁^{spCFP/YFP} stably expressed in HEK-293 cells (data not shown).

Signals recorded from single HEK-293 cells expressing σ₁^{spCFP/YFP} were then analyzed at emissions of 480 nm (CFP) and 535 nm (YFP) upon excitation at 436 nm (CFP excitation). Thus, in Figure 2A emission intensities of YFP (535 nm, yellow) and CFP (480 nm, cyan) were recorded simultaneously from single cells expressing σ₁^{spCFP/YFP} using fluorescence microscopy. The microscopy illumination allowed photobleaching experiments to verify that the emission at 535 nm was indeed due to intramolecular FRET. Thus, after bleaching of the acceptor fluorophore (i.e. YFP) in the σ₁^{spCFP/YFP} construct by intense light illumination at 500 nm during 5 min, the emission at 480 nm increased and the 535 nm emission decreased, as expected **(****Figure 2A****).** In addition, when cells were observed in the microscope before (pre) and after (post) the photobleaching, it was clear that the overall fluorescence of the donor (i.e. CFP) increased along the cell after applying the photobleaching protocol **(****Figure 2B****).** Next, as additional controls, at comparable fluorescence levels, we also determined FRET efficiency by co-expressing CFP plus σ₁^{YFP} and YFP plus σ₁^{CFP}. Thus, under these experimental conditions, while the FRET efficiency of the corresponding negative controls (i.e. CFP/σ₁^{YFP} and YFP/σ₁^{CFP} pairs) was negligible, the FRET efficiency of the σ₁^{spCFP/YFP} construct was 6.8 ±1.1% (P<0.01) **(****Figure 2C****).** The application of the photobleaching protocol to cells expressing only σ₁^{CFP} did not modify the emission intensity of CFP, as expected (data not shown). Interestingly, under these experimental conditions, when we determined FRET efficiency by co-expressing σ₁^{CFP} plus σ₁^{CFP} we found a significant resonance energy transfer between these two σ₁ constructs (3.5 ± 0.7%; P<0.05) **(****Figure 2C****),** suggesting that σ₁ is able to form homodimers. Overall, these results demonstrate that σ₁^{spCFP/YFP} exhibits a significant intramolecular FRET and also that σ₁ forms stable and specific homodimers when expressed in living cells.

Next, we investigated whether σ₁ challenge produced any rearrangements of the N- and C-terminal domains (i.e. conformational change) of the σ₁ receptor by monitoring real time changes in the FRET efficiency. To this end, changes in the emission intensities of YFP (535 nm, yellow), CFP (480 nm, cyan) and the ratio F^{*}₅₃₅/F^{*}₄₈₀ (black) were recorded simultaneously from single cells after rapid superfusion with σ₁ receptor ligands. The effects of the agonist PRE-048 and the antagonist haloperidol on the FRET signal of σ₁^{spCFP/YFP} were investigated and measured as the bleedthrough-corrected emission intensity ratio F^{*}₅₃₅/F^{*}₄₈₀. Interestingly, the addition of 100 µM PRE-048 produced a rapid decrease in the *F^{*}*₅₃₅/*F^{*}*₄₈₀ ratio **(****Figure 2D****).** After a short delay (-5 s), a monoexponential time course with a time constant τ = 10.3 ±0.3 s (*n* = 3) followed. The symmetrical increase in CFP emission and decrease in YFP emission indicate that the change was due to a decrease in FRET. Under the same experimental conditions, the addition of 100 µM haloperidol produced after a short delay (-2 s) a rapid increase of the *F*^{*}₅₃₅/*F*^{*}₄₈₀ ratio **(****Figure 2E****).** Similarly, a monoexponential time course with a time constant τ = 27.2 ±0.8 s (*n* = 3) followed the haloperidol-mediated FRET increase. Finally, control experiments with coexpression of σ₁^{YFP} and CFP showed no FRET in the absence or in the presence of either PRE-048 or haloperidol (data not shown). Overall, these results demonstrate that the σ₁^{spCFP/YFP} construct undergoes conformational changes upon ligand incubation and that these changes might be opposite depending on the nature of the ligand (i.e. agonist or antagonist).

Finally, several agonists (i.e. Dextromethorphan, (+)-3-PPP, (+)-Pentazocine, (+)-SKF-10,047, DTG and PRE-048) and antagonists (i.e. Haloperidol, E92 and PRE-048) were tested to confirm that the canonical σ₁ agonism and antagonism produced a decrease or an increase in the FRET signal of the σ₁^{spCFP/YFP} respectively **(****Figure 3).** Figure 3 shows representative YFP emission traces recorded from a single HEK-293 cell expressing the σ₁^{spCFP/YFP}, which has been incubated with σ₁ receptor agonists (Dextromethorphan, (+)-3-PPP, (+)-Pentazocine, (+)-SKF-10,047, DTG and PRE-048) and antagonists (Haloperidol, E92 and NE-100). Interestingly, by monitoring the σ₁^{spCFP/YFP} intramolecular FRET signal throughout σ₁ challenge, we observed that agonist and antagonist induced opposite FRET signals in the σ₁^{spCFP/YFP} construct **(****Figure 3****).** Thus, these results confirm that σ₁ receptor ligands produce opposite conformational changes in the σ₁ receptor depending on the nature of the ligand (e.g. agonist vs. antagonist) and, therefore, the σ₁^{spCFP/YFP} construct is a powerful tool to classify σ₁ receptor ligands.

## Claims

1. A fusion protein comprising
(i) a σ₁ receptor or a functionally equivalent variant thereof,
(ii) a donor fluorescent protein moiety, and
(iii) an acceptor fluorescent protein moiety,
wherein said donor and acceptor fluorescent protein moiety are capable of producing Fluorescence Resonance Energy Transfer (FRET), and wherein the σ₁ receptor is flanked by the donor fluorescent protein moiety and the acceptor fluorescent protein moiety.

2. The fusion protein according to claim 1, wherein energy transfer from the donor fluorescent protein moiety in an excited state to the acceptor fluorescent protein moiety increases if a σ₁ receptor antagonist binds to the σ₁ receptor.

3. The fusion protein according to any one of claims 1 to 2, wherein energy transfer from the donor fluorescent protein moiety in an excited state to the acceptor fluorescent protein moiety occurs if no ligand is bound to the σ₁ receptor.

4. The fusion protein according to claim 3, wherein energy transfer from the donor fluorescent protein moiety in an excited state to the acceptor fluorescent protein moiety decreases if a σ₁ receptor agonist binds to the σ₁ receptor.

5. The fusion protein according to any one of claims 1 to 4, wherein the donor fluorescent protein moiety and/or the acceptor fluorescent protein moiety are Aequorea-related fluorescent proteins and, preferably, wherein the donor fluorescent protein moiety is the cyan fluorescent protein (CFP) and/or the acceptor fluorescent protein moiety is the yellow fluorescent protein (YFP).

6. The fusion protein according to any one of claims 1 to 5, further comprising an N-terminal or C-terminal localization signal peptide.

7. The fusion protein according to any one of claims 1 to 6, wherein said fusion protein has the amino acid sequence according to SEQ NO: 1 or SEQ ID NO: 2.

8. A membrane comprising the fusion protein as defined in any one of claims 1 to 7.

9. A polynucleotide comprising a coding sequence encoding a fusion protein according to any one of claims 1 to 7.

10. A cell comprising the fusion protein according to any one of claims 1 to 7, the membrane of claim 7, and/or the polynucleotide according to claim 8.

11. Use of a fusion protein according to claims 1-7, a membrane according to claim 8, a polynucleotide according to claim 9 and/or a cell according to claim 10 for identifying a ligand for a σ₁ receptor.

12. The use according to claim 11, wherein said ligand is an antagonist or an agonist.

13. A method for identifying a ligand for a σ₁ receptor comprising:
(i) exposing a fusion protein according to any one of claims 1 to 7, a fusion protein comprised in a membrane according to claim 8, or a fusion protein comprised in a cell according to claim 10 to light of a wavelength exciting the donor fluorescent protein moiety, upon which the donor fluorescent protein moiety fluoresces at a first emission wavelength, wherein the light of the first emission length is capable of exciting the acceptor fluorescent protein moiety;
(ii) detecting light of a second emission wavelength emitted by the acceptor protein moiety;
(iii) contacting the fusion protein, membrane and/or cell of step (i) with a test compound; and
(iv) detecting light of a second emission wavelength emitted by the acceptor protein moiety of the fusion protein of step (iii) and, optionally, a control;
wherein, if the intensity of light of a second emission wavelength emitted during step (i) is known or if a control is included, step (ii) may be omitted and, when omitted, step (iii) may be carried out before step (i), and wherein the test compound is identified as a ligand for a σ₁ receptor if there is a difference in the intensity of light of the second emission wavelength detected in step (iv) and
- the intensity of light of the second emission wavelength detected in step (ii),
- the intensity of light of the second emission wavelength known to be emitted during step (i), or
- the intensity of light of the second emission wavelength of the control.

14. The method of claim 13, comprising:
(i) exposing a fusion protein according to any one of claims 1-7, a fusion protein comprised in a membrane according to claim 8, or a fusion protein comprised in a cell according to claim 10 to light of a wavelength exciting the donor fluorescent protein moiety, upon which the donor fluorescent protein moiety fluoresces at a first emission wavelength, wherein the light of the first emission length is capable of exciting the acceptor fluorescent protein moiety;
(ii) detecting light of a second emission wavelength emitted by the acceptor protein moiety of said fusion protein;
(iii) contacting said fusion protein with a test compound; and
(iv) detecting light of a second emission wavelength emitted by the acceptor protein moiety of said fusion protein;
wherein the test compound is identified as a ligand for a σ₁ receptor if there is a difference in the intensity of light of the second emission wavelength detected in step (iv) and the intensity of light of the second emission wavelength detected in step (ii).

15. A kit comprising the fusion protein according to any one of claims 1 to 7, the membrane of claim 8, the polynucleotide according to claim 9, the cell according to claim 10, and/or reagents for carrying out the method of claims 13-14.


SEQUENCE LISTING

<110>  Laboratorios del Dr. Esteve, S.A.

<120>  METHOD TO IDENTIFY LIGANDS FOR SIGMA-1 RECEPTORS

<130>  P7413EP00

<160>  16

<170>  PatentIn version 3.5

<210>  1
<211>  710
<212>  PRT
<213>  Artificial Sequence

<220>
<223>  Fusion Protein without signal peptide

<400>  1

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Trp Gly Val Gln Cys Phe Ala Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Ala Ile Ser Asp Asn Val Tyr Ile Thr Ala Asp Lys Gln Lys Asn
145                 150                 155                 160

Gly Ile Lys Ala Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Lys Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys Glu
225                 230                 235                 240

Phe Met Gln Trp Ala Val Gly Arg Arg Trp Ala Trp Ala Ala Leu Leu
                245                 250                 255

Leu Ala Val Ala Ala Val Leu Thr Gln Val Val Trp Leu Trp Leu Gly
            260                 265                 270

Thr Gln Ser Phe Val Phe Gln Arg Glu Glu Ile Ala Gln Leu Ala Arg
        275                 280                 285

Gln Tyr Ala Gly Leu Asp His Glu Leu Ala Phe Ser Arg Leu Ile Val
    290                 295                 300

Glu Leu Arg Arg Leu His Pro Gly His Val Leu Pro Asp Glu Glu Leu
305                 310                 315                 320

Gln Trp Val Phe Val Asn Ala Gly Gly Trp Met Gly Ala Met Cys Leu
                325                 330                 335

Leu His Ala Ser Leu Ser Glu Tyr Val Leu Leu Phe Gly Thr Ala Leu
            340                 345                 350

Gly Ser Arg Gly His Ser Gly Arg Tyr Trp Ala Glu Ile Ser Asp Thr
        355                 360                 365

Ile Ile Ser Gly Thr Phe His Gln Trp Arg Glu Gly Thr Thr Lys Ser
    370                 375                 380

Glu Val Phe Tyr Pro Gly Glu Thr Val Val His Gly Pro Gly Glu Ala
385                 390                 395                 400

Thr Ala Val Glu Trp Gly Pro Asn Thr Trp Met Val Glu Tyr Gly Arg
                405                 410                 415

Gly Val Ile Pro Ser Thr Leu Ala Phe Ala Leu Ala Asp Thr Val Phe
            420                 425                 430

Ser Thr Gln Asp Phe Leu Thr Leu Phe Tyr Thr Leu Arg Ser Tyr Ala
        435                 440                 445

Arg Gly Leu Arg Leu Glu Leu Thr Thr Tyr Leu Phe Gly Gln Asp Pro
    450                 455                 460

Arg Asp Pro Pro Val Ala Thr Met Val Ser Lys Gly Glu Glu Leu Phe
465                 470                 475                 480

Thr Gly Val Val Pro Ile Leu Val Glu Leu Asp Gly Asp Val Asn Gly
                485                 490                 495

His Lys Phe Ser Val Ser Gly Glu Gly Glu Gly Asp Ala Thr Tyr Gly
            500                 505                 510

Lys Leu Thr Leu Lys Leu Ile Cys Thr Thr Gly Lys Leu Pro Val Pro
        515                 520                 525

Trp Pro Thr Leu Val Thr Thr Phe Gly Tyr Gly Leu Gln Cys Phe Ala
    530                 535                 540

Arg Tyr Pro Asp His Met Lys Gln His Asp Phe Phe Lys Ser Ala Met
545                 550                 555                 560

Pro Glu Gly Tyr Val Gln Glu Arg Thr Ile Phe Phe Lys Asp Asp Gly
                565                 570                 575

Asn Tyr Lys Thr Arg Ala Glu Val Lys Phe Glu Gly Asp Thr Leu Val
            580                 585                 590

Asn Arg Ile Glu Leu Lys Gly Ile Asp Phe Lys Glu Asp Gly Asn Ile
        595                 600                 605

Leu Gly His Lys Leu Glu Tyr Asn Tyr Asn Ser His Asn Val Tyr Ile
    610                 615                 620

Met Ala Asp Lys Gln Lys Asn Gly Ile Lys Val Asn Phe Lys Ile Arg
625                 630                 635                 640

His Asn Ile Glu Asp Gly Ser Val Gln Leu Ala Asp His Tyr Gln Gln
                645                 650                 655

Asn Thr Pro Ile Gly Asp Gly Pro Val Leu Leu Pro Asp Asn His Tyr
            660                 665                 670

Leu Ser Tyr Gln Ser Ala Leu Ser Lys Asp Pro Asn Glu Lys Arg Asp
        675                 680                 685

His Met Val Leu Leu Glu Phe Val Thr Ala Ala Gly Ile Thr Leu Gly

690                 695                 700

Met Asp Glu Leu Tyr Lys
705                 710

<210>  2
<211>  735
<212>  PRT
<213>  Artificial Sequence

<220>
<223>  Fusion Protein with signal peptide

<400>  2

Met Gly Thr Ala Arg Ile Ala Pro Gly Leu Ala Leu Leu Leu Cys Cys
1               5                   10                  15

Pro Val Leu Ser Ser Ala Tyr Ala Leu Ala Val Ser Lys Gly Glu Glu
            20                  25                  30

Leu Phe Thr Gly Val Val Pro Ile Leu Val Glu Leu Asp Gly Asp Val
        35                  40                  45

Asn Gly His Lys Phe Ser Val Ser Gly Glu Gly Glu Gly Asp Ala Thr
    50                  55                  60

Tyr Gly Lys Leu Thr Leu Lys Phe Ile Cys Thr Thr Gly Lys Leu Pro
65                  70                  75                  80

Val Pro Trp Pro Thr Leu Val Thr Thr Leu Thr Trp Gly Val Gln Cys
                85                  90                  95

Phe Ala Arg Tyr Pro Asp His Met Lys Gln His Asp Phe Phe Lys Ser
            100                 105                 110

Ala Met Pro Glu Gly Tyr Val Gln Glu Arg Thr Ile Phe Phe Lys Asp
        115                 120                 125

Asp Gly Asn Tyr Lys Thr Arg Ala Glu Val Lys Phe Glu Gly Asp Thr
    130                 135                 140

Leu Val Asn Arg Ile Glu Leu Lys Gly Ile Asp Phe Lys Glu Asp Gly
145                 150                 155                 160

Asn Ile Leu Gly His Lys Leu Glu Tyr Asn Ala Ile Ser Asp Asn Val
                165                 170                 175

Tyr Ile Thr Ala Asp Lys Gln Lys Asn Gly Ile Lys Ala Asn Phe Lys
            180                 185                 190

Ile Arg His Asn Ile Glu Asp Gly Ser Val Gln Leu Ala Asp His Tyr
        195                 200                 205

Gln Gln Asn Thr Pro Ile Gly Asp Gly Pro Val Leu Leu Pro Asp Asn
    210                 215                 220

His Tyr Leu Ser Thr Gln Ser Lys Leu Ser Lys Asp Pro Asn Glu Lys
225                 230                 235                 240

Arg Asp His Met Val Leu Leu Glu Phe Val Thr Ala Ala Gly Ile Thr
                245                 250                 255

Leu Gly Met Asp Glu Leu Tyr Lys Glu Phe Met Gln Trp Ala Val Gly
            260                 265                 270

Arg Arg Trp Ala Trp Ala Ala Leu Leu Leu Ala Val Ala Ala Val Leu
        275                 280                 285

Thr Gln Val Val Trp Leu Trp Leu Gly Thr Gln Ser Phe Val Phe Gln
    290                 295                 300

Arg Glu Glu Ile Ala Gln Leu Ala Arg Gln Tyr Ala Gly Leu Asp His
305                 310                 315                 320

Glu Leu Ala Phe Ser Arg Leu Ile Val Glu Leu Arg Arg Leu His Pro
                325                 330                 335

Gly His Val Leu Pro Asp Glu Glu Leu Gln Trp Val Phe Val Asn Ala
            340                 345                 350

Gly Gly Trp Met Gly Ala Met Cys Leu Leu His Ala Ser Leu Ser Glu
        355                 360                 365

Tyr Val Leu Leu Phe Gly Thr Ala Leu Gly Ser Arg Gly His Ser Gly
    370                 375                 380

Arg Tyr Trp Ala Glu Ile Ser Asp Thr Ile Ile Ser Gly Thr Phe His
385                 390                 395                 400

Gln Trp Arg Glu Gly Thr Thr Lys Ser Glu Val Phe Tyr Pro Gly Glu
                405                 410                 415

Thr Val Val His Gly Pro Gly Glu Ala Thr Ala Val Glu Trp Gly Pro
            420                 425                 430

Asn Thr Trp Met Val Glu Tyr Gly Arg Gly Val Ile Pro Ser Thr Leu
        435                 440                 445

Ala Phe Ala Leu Ala Asp Thr Val Phe Ser Thr Gln Asp Phe Leu Thr

450                 455                 460

Leu Phe Tyr Thr Leu Arg Ser Tyr Ala Arg Gly Leu Arg Leu Glu Leu
465                 470                 475                 480

Thr Thr Tyr Leu Phe Gly Gln Asp Pro Arg Asp Pro Pro Val Ala Thr
                485                 490                 495

Met Val Ser Lys Gly Glu Glu Leu Phe Thr Gly Val Val Pro Ile Leu
            500                 505                 510

Val Glu Leu Asp Gly Asp Val Asn Gly His Lys Phe Ser Val Ser Gly
        515                 520                 525

Glu Gly Glu Gly Asp Ala Thr Tyr Gly Lys Leu Thr Leu Lys Leu Ile
    530                 535                 540

Cys Thr Thr Gly Lys Leu Pro Val Pro Trp Pro Thr Leu Val Thr Thr
545                 550                 555                 560

Phe Gly Tyr Gly Leu Gln Cys Phe Ala Arg Tyr Pro Asp His Met Lys
                565                 570                 575

Gln His Asp Phe Phe Lys Ser Ala Met Pro Glu Gly Tyr Val Gln Glu
            580                 585                 590

Arg Thr Ile Phe Phe Lys Asp Asp Gly Asn Tyr Lys Thr Arg Ala Glu
        595                 600                 605

Val Lys Phe Glu Gly Asp Thr Leu Val Asn Arg Ile Glu Leu Lys Gly
    610                 615                 620

Ile Asp Phe Lys Glu Asp Gly Asn Ile Leu Gly His Lys Leu Glu Tyr
625                 630                 635                 640

Asn Tyr Asn Ser His Asn Val Tyr Ile Met Ala Asp Lys Gln Lys Asn
                645                 650                 655

Gly Ile Lys Val Asn Phe Lys Ile Arg His Asn Ile Glu Asp Gly Ser
            660                 665                 670

Val Gln Leu Ala Asp His Tyr Gln Gln Asn Thr Pro Ile Gly Asp Gly
        675                 680                 685

Pro Val Leu Leu Pro Asp Asn His Tyr Leu Ser Tyr Gln Ser Ala Leu
    690                 695                 700

Ser Lys Asp Pro Asn Glu Lys Arg Asp His Met Val Leu Leu Glu Phe
705                 710                 715                 720

Val Thr Ala Ala Gly Ile Thr Leu Gly Met Asp Glu Leu Tyr Lys
                725                 730                 735

<210>  3
<211>  2133
<212>  DNA
<213>  Artificial Sequence

<220>
<223>  Polynucleotide encoding fusion protein without signal peptide

<400>  3
atggtgagca agggcgagga gctgttcacc ggggtggtgc ccatcctggt cgagctggac      60

ggcgacgtaa acggccacaa gttcagcgtg tccggcgagg gcgagggcga tgccacctac     120

ggcaagctga ccctgaagtt catctgcacc accggcaagc tgcccgtgcc ctggcccacc     180

ctcgtgacca ccctgacctg gggcgtgcag tgcttcgccc gctaccccga ccacatgaag     240

cagcacgact tcttcaagtc cgccatgccc gaaggctacg tccaggagcg caccatcttc     300

ttcaaggacg acggcaacta caagacccgc gccgaggtga agttcgaggg cgacaccctg     360

gtgaaccgca tcgagctgaa gggcatcgac ttcaaggagg acggcaacat cctggggcac     420

aagctggagt acaacgccat cagcgacaac gtctatatca ccgccgacaa gcagaagaac     480

ggcatcaagg ccaacttcaa gatccgccac aacatcgagg acggcagcgt gcagctcgcc     540

gaccactacc agcagaacac ccccatcggc gacggccccg tgctgctgcc cgacaaccac     600

tacctgagca cccagtccaa gctgagcaaa gaccccaacg agaagcgcga tcacatggtc     660

ctgctggagt tcgtgaccgc cgccgggatc actctcggca tggacgagct gtacaaggaa     720

ttcatgcagt gggccgtggg ccggcggtgg gcgtgggccg cgctgctcct ggctgtcgca     780

gcggtgctga cccaggtcgt ctggctctgg ctgggtacgc agagcttcgt cttccagcgc     840

gaagagatag cgcagttggc gcggcagtac gctgggctgg accacgagct ggccttctct     900

cgtctgatcg tggagctgcg gcggctgcac ccaggccacg tgctgcccga cgaggagctg     960

cagtgggtgt tcgtgaatgc gggtggctgg atgggcgcca tgtgccttct gcacgcctcg    1020

ctgtccgagt atgtgctgct cttcggcacc gccttgggct cccgcggcca ctcggggcgc    1080

tactgggctg agatctcgga taccatcatc tctggcacct tccaccagtg gagagagggc    1140

accaccaaaa gtgaggtctt ctacccaggg gagacggtag tacacgggcc tggtgaggca    1200

acagctgtgg agtgggggcc aaacacatgg atggtggagt acggccgggg cgtcatccca    1260

tccaccctgg ccttcgcgct ggccgacact gtcttcagca cccaggactt cctcaccctc    1320

ttctatactc ttcgctccta tgctcggggc ctccggcttg agctcaccac ctacctcttt    1380

ggccaggacc ctcgggatcc accggtcgcc accatggtga gcaagggcga ggagctgttc    1440

accggggtgg tgcccatcct ggtcgagctg gacggcgacg taaacggcca caagttcagc    1500

gtgtccggcg agggcgaggg cgatgccacc tacggcaagc tgaccctgaa gcttatctgc 1560

accaccggca agctgcccgt gccctggccc accctcgtga ccaccttcgg ctacggcctg 1620

cagtgcttcg cccgctaccc cgaccacatg aagcagcacg acttcttcaa gtccgccatg 1680

cccgaaggct acgtccagga gcgcaccatc ttcttcaagg acgacggcaa ctacaagacc 1740

cgcgccgagg tgaagttcga gggcgacacc ctggtgaacc gcatcgagct gaagggcatc 1800

gacttcaagg aggacggcaa catcctgggg cacaagctgg agtacaacta caacagccac 1860

aacgtctata tcatggccga caagcagaag aacggcatca aggtgaactt caagatccgc 1920

cacaacatcg aggacggcag cgtgcagctc gccgaccact accagcagaa cacccccatc 1980

ggcgacggcc ccgtgctgct gcccgacaac cactacctga gctaccagtc cgccctgagc 2040

aaagacccca acgagaagcg cgatcacatg gtcctgctgg agttcgtgac cgccgccggg 2100

atcactctcg gcatggacga gctgtacaag taa 2133

<210> 4
<211> 2205
<212> DNA
<213> Artificial Sequence

<220>
<223> Polynucleotide encoding FP with signal peptide

<400> 4
atggggaccg cccggatcgc acccggcctg gcgctcctgc tctgctgccc cgtgctcagc 60

tccgcgtacg cgctagccgt gagcaagggc gaggagctgt tcaccggggt ggtgcccatc 120

ctggtcgagc tggacggcga cgtaaacggc cacaagttca gcgtgtccgg cgagggcgag 180

ggcgatgcca cctacggcaa gctgaccctg aagttcatct gcaccaccgg caagctgccc 240

gtgccctggc ccaccctcgt gaccaccctg acctggggcg tgcagtgctt cgcccgctac 300

cccgaccaca tgaagcagca cgacttcttc aagtccgcca tgcccgaagg ctacgtccag 360

gagcgcacca tcttcttcaa ggacgacggc aactacaaga cccgcgccga ggtgaagttc 420

gagggcgaca ccctggtgaa ccgcatcgag ctgaagggca tcgacttcaa ggaggacggc 480

aacatcctgg ggcacaagct ggagtacaac gccatcagcg acaacgttat atcaccgccg 540

acaagcagaa gaacggcatc aaggccaact tcaagatccg ccacaacatc gaggacggca 600

gcgtgcagct cgccgaccac tacagcagaa cacccccatc ggcgacggcc ccgtgctgct 660

gcccgacaac cactacctga gcacccagtc caagctgagc aaagacccca acgagaagcg 720

cgatcacatg gtcctgctgg agttcgtgac cgccgccggg atcactctcg gcatggacga 780

gctgtacaag gaattcatgc agtgggccgt gggccggcgg tgggcgtggg ccgcgctgct 840

cctggctgtc gcagcggtgc tgacccaggt cgtctggctc tggctgggta cgcagagctt 900

cgtcttccag cgcgaagaga tagcgcagtt ggcgcggcag tacgctgggc tggaccacga 960

gctggccttc tctcgtctga tcgtggagct gcggcggctg cacccggcca cgtgctgccc 1020

gacgaggagc tgcagtgggt gttcgtgaat gcgggtggct ggatgggcgc catgtgcctt 1080

ctgcacgcct cgctgtccga gtatgtgctg ctcttcggca ccgccttggg ctcccgcggc 1140

cactcggggc gctactgggc tgagatctcg gataccatca tctctggcac cttccaccag 1200

tggagagagg gcaccaccaa aagtgaggtc ttctacccag gggagacggt agtacacggg 1260

cctggtgagg caacagctgt ggagtggggg ccaaacacat ggatggtgga gtacggccgg 1320

ggcgtcatcc catccaccct ggccttcgcg ctggccgaca ctgtcttcag cacccaggac 1380

ttcctcaccc tcttctatac tcttcgctcc tatgctcggg gcctccggct tgagctcacc 1440

acctacctct ttggccagga ccctcgggat ccaccggtcg ccaccatggt gagcaagggc 1500

gaggagctgt tcaccggggt ggtgcccatc ctggtcgagc tggacggcga cgtaaacggc 1560

cacaagttca gcgtgtccgg cgagggcgag ggcgatgcca cctacggcaa gctgaccctg 1620

aagcttatct gcaccaccgg caagctgccc gtgccctggc ccaccctcgt gaccaccttc 1680

ggctacggcc tgcagtgctt cgcccgctac cccgaccaca tgaagcagca cgacttcttc 1740

aagtccgcca tgcccgaagg ctacgtccag gagcgcacca tcttcttcaa ggacgacggc 1800

aactacaaga cccgcgccga ggtgaagttc gagggcgaca ccctggtgaa ccgcatcgag 1860

ctgaagggca tcgacttcaa ggaggacggc aacatcctgg ggcacaagct ggagtacaac 1920

tacaacagcc acaacgtcta tatcatggcc gacaagcaga agaacggcat caaggtgaac 1980

ttcaagatcc gccacaacat cgaggacggc agcgtgcagc tcgccgacca ctaccagcag 2040

aacaccccca tcggcgacgg ccccgtgctg ctgcccgaca accactacct gagctaccag 2100

tccgccctga gcaaagaccc caacgagaag cgcgatcaca tggtcctgct ggagttcgtg 2160

accgccgccg ggatcactct cggcatggac gagctgtaca agtaa 2205

<210> 5
<211> 25
<212> PRT
<213> Homo sapiens

<400> 5

Met Gly Thr Ala Arg Ile Ala Pro Gly Leu Ala Leu Leu Leu Cys Cys
1               5                   10                  15

Pro Val Leu Ser Ser Ala Tyr Ala Leu
            20                  25

<210> 6
<211> 20
<212> PRT
<213> Homo sapiens

<400> 6

Met Val Leu Leu Leu Ile Leu Ser Val Leu Leu Leu Lys Glu Asp Val

1               5                   10                  15

Arg Gly Ser Ala
            20

<210>  7
<211>  38
<212>  PRT
<213>  Homo sapiens

<400>  7

Met Ala Ser Pro Arg Ser Ser Gly Gln Pro Gly Pro Pro Pro Pro Pro
1               5                   10                  15

Pro Pro Pro Pro Ala Arg Leu Leu Leu Leu Leu Leu Leu Pro Leu Leu
            20                  25                  30

Leu Pro Leu Ala Pro Gly
        35

<210>  8
<211>  8
<212>  PRT
<213>  Homo sapiens

<400>  8

Pro Pro Lys Lys Lys Arg Lys Val
1               5

<210>  9
<211>  24
<212>  PRT
<213>  Homo sapiens

<400>  9

Met Leu Ser Leu Arg Gln Ser Ile Arg Phe Phe Lys Pro Ala Thr Arg
1               5                   10                  15

Thr Leu Cys Ser Ser Arg Tyr Leu
            20

<210>  10
<211>  29
<212>  PRT
<213>  Homo sapiens

<400>  10

Met Met Ser Phe Val Ser Leu Leu Leu Val Gly Ile Leu Phe Trp Ala
1               5                   10                  15

Thr Glu Ala Glu Gln Leu Thr Lys Cys Glu Val Phe Gln
            20                  25

<210>  11
<211>  4
<212>  PRT
<213>  Homo sapiens

<400>  11

Lys Asp Glu Leu
1


<210>  12
<211>  21
<212>  DNA
<213>  Artificial Sequence

<220>
<223>  Primer FSEcoRI

<400>  12
cggaattcat gcagtgggcc g                                                21


<210>  13
<211>  28
<212>  DNA
<213>  Artificial Sequence

<220>
<223>  RSBamHI

<400>  13
caggatcccg agggtcctgg ccaaagag                                         28


<210>  14
<211>  30
<212>  DNA
<213>  Artificial Sequence

<220>
<223>  Primer FCFPHindIII

<400>  14
cgaagttcat ggtgagcaag ggcgaggagc                                       30


<210>  15
<211>  30
<212>  DNA
<213>  Artificial Sequence

<220>
<223>  Primer RCFPEcoRI

<400>  15
ccggaattcc ttgtacagct cgtccatgcc                                       30


<210>  16
<211>  18
<212>  DNA
<213>  Artificial Sequence

<220>
<223>  Primer FspCFPHindIII

<400>  16
gtttaaactt aagttcgg                                                    18